# EUROPEAN PATENT APPLICATION

(11) **EP 4 509 613 A1**
(43) Date of publication of application: **19.02.2025**
(21) Application number: 23788425.9
(22) Date of filing: 14.04.2023
(51) Int. Cl.: C12P 21/02, C07K 14/50, C07K 19/00, C12N 1/21, C12N 5/07, C12N 5/077, C12N 15/18, C12N 15/62

(54) **METHOD FOR PRODUCING CULTURED MEAT**

(30) Priority: 15.04.2022 JP 2022067537
(71) Applicant: AJINOMOTO CO., INC., Chuo-ku Tokyo 104-8315 (JP)
(72) Inventor: TSUYOSHI, Naoko, Kawasaki-shi, Kanagawa 210-8681 (JP); MANNEN, Teruhisa, Kawasaki-shi, Kanagawa 210-8681 (JP); MATSUDA, Yoshihiko, Kawasaki-shi, Kanagawa 210-8681 (JP); OGAWA, Shimpei, Kawasaki-shi, Kanagawa 210-8681 (JP); KOSEKI, Yasumichi, Kawasaki-shi, Kanagawa 210-8681 (JP); KADO, Tomomi, Kawasaki-shi, Kanagawa 210-8681 (JP)
(74) Representative: Hoffmann Eitle
(86) International application number: PCT/JP2023/015195
(87) International publication number: WO 2023/200008

(57) **Abstract**

A method of producing cultured meat is provided. Cultured meat is produced by culturing animal cells in the presence of bovine basic fibroblast growth factor (bbFGF) produced by using a coryneform bacterium as an expression host.

## Description

### TECHNICAL FIELD

The present invention relates to a method of producing cultured meat.

### BACKGROUND ART

In recent years, technologies for producing cultured meat have been attracting attention. One of the challenges in producing cultured meat is the high production cost.

Growth factors such as bovine basic fibroblast growth factor (bbFGF) are typically purified to a high purity with chromatography or other means and used in cell culture applications in regenerative medicine and so forth.

### SUMMARY OF THE INVENTION

### PROBLEMS TO BE ACHIEVED BY THE INVENTION

An object of the present invention is to provide a method of producing cultured meat.

### MEANS FOR SOLVING THE PROBLEMS

The inventors of the present invention diligently conducted researches to achieve the above object, as a result, found that bovine basic fibroblast growth factor (bbFGF) expressed by a coryneform bacterium as a host is effective for the production of cultured meat, and thus accomplished the present invention.

Accordingly, the present invention can be illustrated as follows.
[1] A method of producing bovine basic fibroblast growth factor (bbFGF) for production of cultured meat, the method comprising:
   a step of culturing a coryneform bacterium having a gene construct for secretory expression of bbFGF to obtain a culture product containing bbFGF.
[2] The method described above, further comprising a step of removing cells of the coryneform bacterium from the culture product.
[3] The method described above, not comprising a step of purifying bbFGF.
[4] The method described above, not comprising a step of purifying bbFGF with chromatography.
[5] The method described above, wherein the gene construct contains a promoter sequence that functions in the coryneform bacterium, a nucleic acid sequence encoding a signal peptide that functions in the coryneform bacterium, and a nucleic acid sequence encoding bbFGF in the direction from 5' to 3', and
   wherein the bbFGF is expressed as a fusion protein with the signal peptide.
[6] The method described above, wherein the coryneform bacterium is a bacterium belonging to the genus *Corynebacterium.*
[7] The method described above, wherein the coryneform bacterium is *Corynebacterium glutamicum.*
[8] A method of producing cultured meat, comprising:
   a step of culturing animal cells in the presence of bovine basic fibroblast growth factor (bbFGF),
   wherein the bbFGF is produced by the method described above.

### BRIEF DESCRIPTION OF THE DRAWINGS

Figure 1 shows a photograph showing the results of SDS-PAGE analysis of bbFGF expressed and secreted (secretory expression) by *C. glutamicum* used as a host.
Figure 2 shows a diagram showing a chromatogram obtained when bbFGF expressed and secreted by *C. glutamicum* used as a host was separated with cation exchange chromatography.
Figure 3 shows a diagram showing a chromatogram obtained when bbFGF expressed and secreted by *C. glutamicum* used as a host was separated with cation exchange chromatography.
Figure 4 shows a graph showing proliferation of bovine muscle stem cells in a serum-free medium supplemented with bbFGF expressed and secreted by *C. glutamicum* used as a host. The data (vertical axis) are shown as relative values based on the number of the cells at the start of the culture, which is taken as 1.
Figure 5 shows a graph showing proliferation rate of bovine muscle stem cells in a serum-free medium supplemented with bbFGF expressed and secreted by *C. glutamicum* used as a host. The group numbers are shown under the horizontal axis.
Figure 6 shows a graph showing doubling time of bovine muscle stem cells in a serum-free medium supplemented with bbFGF expressed and secreted by *C. glutamicum* used as a host. The group numbers are shown under the horizontal axis.
Figure 7 shows a graph showing proliferation of bovine muscle stem cells in a serum-free medium supplemented with bbFGF expressed and secreted by *C. glutamicum* used as a host. The data (vertical axis) are shown as relative values based on the number of the cells at the start of the culture, which is taken as 1.
Figure 8 shows a graph showing proliferation rate of bovine muscle stem cells in a serum-free medium supplemented with bbFGF expressed and secreted by *C. glutamicum* used as a host. The group numbers are shown under the horizontal axis.
Figure 9 shows a graph showing doubling time of bovine muscle stem cells in a serum-free medium supplemented with bbFGF expressed and secreted by *C. glutamicum* used as a host. The group numbers are shown under the horizontal axis.

### MODE FOR CARRYING OUT THE INVENTION

Hereafter, the present invention will be explained in detail.

### 1. Active ingredient

In the present invention, bovine basic fibroblast growth factor (bbFGF) produced by using a coryneform bacterium as an expression host is utilized as an active ingredient. The bbFGF produced by using a coryneform bacterium as an expression host is also referred to as the "active ingredient".

By utilizing the active ingredient, specifically, by culturing animal cells in the presence of the active ingredient, proliferation of the animal cells can be promoted, i.e., the effect of promoting proliferation of animal cells can be obtained. This effect is also referred to as the "proliferation-promoting effect". The proliferation-promoting effect can be confirmed by, for example, culturing animal cells in the presence and absence of the active ingredient, and comparing the degrees of proliferation of the animal cells. By promoting proliferation of animal cells, for example, cultured meat can be efficiently produced. That is, the active ingredient may be for the promotion of animal cell proliferation, specifically, for the production of cultured meat.

Hereafter, a method of producing the active ingredient using a coryneform bacterium as an expression host will be explained.

The active ingredient can be produced by culturing a coryneform bacterium having a gene construct for secretory expression of bbFGF. That is, the method of producing the active ingredient may be a method comprising a step of culturing a coryneform bacterium having a gene construct for secretory expression of bbFGF. This step is also referred to as the "coryneform bacterium-culturing step". By the coryneform bacterium-culturing step, bbFGF is produced and secreted (produced through secretory production), and thus a culture product containing bbFGF (specifically, a culture product containing bbFGF produced and secreted) is obtained. Therefore, the coryneform bacterium-culturing step may specifically be a step of producing bbFGF through secretory production by culturing a coryneform bacterium having a gene construct for secretory expression of bbFGF. The coryneform bacterium-culturing step may also specifically be a step of culturing a coryneform bacterium having a gene construct for secretory expression of bbFGF to obtain a culture product containing bbFGF.

The above coryneform bacterium (i.e., coryneform bacterium used in the production of the active ingredient) is also referred to as "the bacterium of the present invention". The bacterium of the present invention or a parent strain used to construct it is also referred to as the "host".

The above gene construct (i.e., a gene construct for secretory expression of bbFGF) is also referred to "bbFGF expression cassette".

### 1-1. Bacterium of the present invention

The bacterium of the present invention is a coryneform bacterium that has a gene construct for secretory expression of bbFGF (i.e., a bbFGF expression cassette).

The bacterium of the present invention has an ability to produce and secrete bbFGF (secretory production). The bacterium of the present invention has an ability to produce and secrete bbFGF relying on at least having the bbFGF expression cassette.

The expression that bbFGF is "secreted" means that bbFGF is transferred to the outside of bacterial cell (extracellular region). Examples of the extracellular region of bacterial cell include the medium and the cell surface layer. That is, secreted bbFGF may be present in the medium, in the cell surface layer, or in both of the medium and the cell surface layer. That is, "secretion" of bbFGF is not limited to the case where all the molecules of bbFGF are finally completely in free state in the medium, but also encompasses, for example, the case where all the molecules of bbFGF are present in the bacterial cell surface layers, or the case where some molecules of bbFGF are present in the medium and the rest in the bacterial cell surface layers.

That is, the "ability to produce and secrete bbFGF" means an ability of the bacterium of the present invention to secrete bbFGF into the medium and/or the bacterial cell surface layers and to accumulate it to such an extent that it can be recovered from the medium and/or the bacterial cell surface layers, when the bacterium is cultured in a medium. The accumulation amount may be, for example, preferably 10 µg/L or more, more preferably 1 mg/L or more, especially preferably 100 mg/L or more, further preferably 1 g/L or more, in terms of the accumulation amount in the medium. The accumulation amount may be, for example, in terms of accumulation amount in the bacterial cell surface layers, such an amount that when bbFGF in the bacterial cell surface layers is collected and suspended in the same volume of liquid as that of the medium, the concentration of bbFGF in the suspension is preferably 10 µg/L or more, more preferably 1 mg/L or more, especially preferably 100 mg/L or more.

Coryneform bacteria are aerobic Gram-positive rods. Examples of coryneform bacteria include bacteria belonging to the genus *Corynebacterium,* bacteria belonging to the genus *Brevibacterium,* bacteria belonging to the genus *Microbacterium,* and so forth. Examples of the advantages of using coryneform bacteria include that they originally secrete very few proteins outside the bacterial cells compared with filamentous fungi, yeasts, *Bacillus* bacteria, etc. conventionally used for secretory production of proteins, and thus it is expected that the purification step can be simplified or omitted when a protein is produced through secretory production, that they grow well in a simple medium containing sugar, ammonia, inorganic salts, etc., and thus are superior in terms of medium cost, simplicity of culture method, and culture productivity, and so forth.

Specific examples of the coryneform bacteria include the following species.
*Corynebacterium acetoacidophilum*
*Corynebacterium acetoglutamicum*
*Corynebacterium alkanolyticum*
*Corynebacterium callunae*
*Corynebacterium casei*
*Corynebacterium crenatum*
*Corynebacterium flavescens*
*Corynebacterium glutamicum*
*Corynebacterium lilium*
*Corynebacterium melassecola*
*Corynebacterium thermoaminogenes* (*Corynebacterium efficiens*)
*Corynebacterium herculis*
*Brevibacterium casei*
*Brevibacterium divaricatum* (*Corynebacterium glutamicum*)
*Brevibacterium flavum* (*Corynebacterium glutamicum*)
*Brevibacterium immariophilum*
*Brevibacterium lactofermentum* (*Corynebacterium glutamicum*)
*Brevibacterium roseum*
*Brevibacterium saccharolyticum*
*Brevibacterium thiogenitalis*
*Corynebacterium ammoniagenes* (*Corynebacterium stationis*)
*Brevibacterium album*
*Brevibacterium cerinum*
*Microbacterium ammoniaphilum*

Specific examples of the coryneform bacteria include the following strains.
*Corynebacterium acetoacidophilum* ATCC 13870
*Corynebacterium acetoglutamicum* ATCC 15806
*Corynebacterium alkanolyticum* ATCC 21511
*Corynebacterium callunae* ATCC 15991
*Corynebacterium casei* JCM 12072
*Corynebacterium crenatum* AS 1.542
*Corynebacterium flavescens* ATCC 10340
*Corynebacterium glutamicum* ATCC 13020, ATCC 13032, ATCC 13060, ATCC 13869, and FERM BP-734
*Corynebacterium lilium* ATCC 15990
*Corynebacterium melassecola* ATCC 17965
*Corynebacterium efficiens* (*Corynebacterium thermoaminogenes*) AJ12340 (FERM BP-1539)
*Corynebacterium herculis* ATCC 13868
*Brevibacterium casei* ATCC 35513
*Brevibacterium divaricatum* (*Corynebacterium glutamicum*) ATCC 14020
*Brevibacterium flavum* (*Corynebacterium glutamicum*) ATCC 13826, ATCC 14067, and AJ12418 (FERM BP-2205)
*Brevibacterium immariophilum* ATCC 14068
*Brevibacterium lactofermentum* (*Corynebacterium glutamicum*) ATCC 13869
*Brevibacterium roseum* ATCC 13825
*Brevibacterium saccharolyticum* ATCC 14066
*Brevibacterium thiogenitalis* ATCC 19240
*Corynebacterium ammoniagenes* (*Corynebacterium stationis*) ATCC 6871 and ATCC 6872
*Brevibacterium album* ATCC 15111
*Brevibacterium cerinum* ATCC 15112
*Microbacterium ammoniaphilum* ATCC 15354

The *Corynebacterium* bacteria also include bacteria that were previously classified in the genus *Brevibacterium* but are now integrated into the genus *Corynebacterium* (Int. J. Syst. Bacteriol., 41, 255 (1991)). *Corynebacterium stationis* also includes bacteria that were previously classified as *Corynebacterium ammoniagenes* but were reclassified as *Corynebacterium stationis* on the basis of 16S rRNA sequencing and other analyses (Int. J. Syst. Evol. Microbiol., 60, 874-879 (2010)).

These strains are available from, for example, the American Type Culture Collection (12301 Parklawn Drive, Rockville, Maryland 20852 P.O. Box 1549, Manassas, VA 20108, United States of America). That is, each strain is assigned a corresponding registration number, and can be distributed by using this registration number (see http://www.atcc.org/). The registration numbers of respective strains are listed in the catalogue of the American Type Culture Collection. These strains can otherwise be obtained from, for example, the depositories where the respective strains were deposited.

In particular, *C. glutamicum* AJ12036 (FERM BP-734), which was isolated from the wild strain *C. glutamicum* ATCC 13869 as a streptomycin (Sm)-resistant mutant, is predicted to have a mutation in the gene responsible for the function concerning protein secretion, shows an extremely high ability to produce and secrete proteins, which is higher by approximately 2 to 3 times that that of the parent strain (wild strain) in terms of the amount to be accumulated under optimal culture conditions, and thus is suitable as the host bacterium (WO2002/081694). AJ12036 was originally deposited on March 26, 1984 at the Institute of Microbial Technology, Agency of Industrial Science and Technology (currently the independent administrative agency, National Institute of Technology and Evaluation, International Patent Organism Depositary (NITE IPOD), Room 120, 2-5-8 Kazusakamatari, Kisarazu-shi, Chiba, Japan, postal code 292-0818) as an international deposit, and assigned the accession number FERM BP-734.

Alternatively, a strain with an enhanced ability to produce and secrete a protein may be selected from strains produced from any of the aforementioned coryneform bacteria used as a parent strain by using a mutation method or genetic recombination method, and used as the host. For example, after ultraviolet irradiation or a treatment with a chemical mutagen such as N-methyl-N'-nitrosoguanidine, strains with an enhanced ability to produce and secrete a protein can be selected.

Furthermore, it is particularly desirable to use a strain obtained from any of the strains mentioned above by such a modification that it should not produce cell surface layer proteins as the host, since such a strain facilitates purification of bbFGF secreted in the medium or in the bacterial cell surface layers. Such a modification can be conducted by introducing a mutation into a coding region of a cell surface layer protein or expression regulatory region on the chromosome using a mutation or genetic recombination method. Examples of coryneform bacteria that have been modified not to produce a cell surface layer protein include *C. glutamicum* YDK010 strain (WO2002/081694), which is a strain deficient in the cell surface layer protein PS2 of *C. glutamicum* AJ12036 (FERM BP-734).

The bacterium of the present invention can be obtained by appropriately modifying any of the coryneform bacteria described above (e.g., introducing the bbFGF expression cassette and optionally introducing other modifications). That is, the bacterium of the present invention may be, for example, a modified strain derived from any of the coryneform bacterium described above. Specifically, the bacterium of the present invention may be, for example, a modified strain derived from *C. glutamicum* AJ12036 (FERM BP-734) or a modified strain derived from *C. glutamicum* ATCC 13869. A modified strain derived from *C. glutamicum* AJ12036 (FERM BP-734) is also a modified strain derived from *C. glutamicum* ATCC 13869. The modifications to construct the bacterium of the present invention can be carried out in any order.

### 1-2. Gene construct for secretory expression of bbFGF and introduction thereof

The bacterium of the present invention has a gene construct for secretory expression of bbFGF (i.e., bbFGF expression cassette).

Secretory proteins are generally known to be translated as preproteins (also called prepeptides) or preproproteins (also called prepropeptides) and then processed into mature proteins. Specifically, secretory proteins are generally translated as preproteins or preproproteins, which are then converted into mature proteins or proproteins by cleavage of a signal peptide, which is a pre-moiety, with a protease (commonly called signal peptidase), and the proproteins become mature proteins after further cleavage of a pro-moiety with a protease. Therefore, in the present invention, the signal peptide may be used for secretory production of bbFGF. Such preprotein and preproprotein of a secretory protein may be collectively referred to as "secretory protein precursor". The term "signal peptide" (also referred to as "signal sequence") refers to an amino acid sequence that is present at the N-terminus of a secretory protein precursor and is not normally present in the corresponding natural mature protein.

The bbFGF expression cassette may contain a promoter sequence that functions in coryneform bacteria, a nucleic acid sequence encoding a signal peptide that functions in coryneform bacteria, and a nucleic acid sequence encoding bbFGF in the direction from 5' to 3'. The nucleic acid sequence encoding a signal peptide may be linked downstream of the promoter sequence so that the signal peptide is expressed under the control by the promoter. The nucleic acid sequence encoding bbFGF should be linked downstream of the nucleic acid sequence encoding the signal peptide so that bbFGF is expressed as a fusion protein with the signal peptide. This fusion protein is also referred to as the "fusion protein of the present invention". In the fusion protein of the present invention, the signal peptide and bbFGF may or may not be adjacent to each other. That is, the case where "bbFGF is expressed as a fusion protein with a signal peptide" is not limited to a case where bbFGF is expressed as a fusion protein with the signal peptide in which bbFGF is adjacent to the signal peptide, but also includes a case where bbFGF is expressed as a fusion protein with the signal peptide in which bbFGF is linked to the signal peptide via another amino acid sequence. For example, as described below, in the fusion protein of the present invention, an insertion sequence such as an amino acid sequence containing Gln-Glu-Thr or an amino acid sequence used for enzymatic cleavage can be contained between the signal peptide and bbFGF. In addition, as described below, bbFGF finally obtained may not have a signal peptide. That is, in order that "bbFGF is expressed as a fusion protein with a signal peptide", it is sufficient if bbFGF constitutes a fusion protein with the signal peptide at the time of expression, and it is not necessary that the finally obtained bbFGF constitutes a fusion protein with the signal peptide. The term nucleic acid sequence may be read as "gene". For example, the nucleic acid sequence encoding bbFGF is also referred to as a "gene encoding bbFGF" or "bbFGF gene". Examples of the nucleic acid sequence includes DNA. The bbFGF expression cassette may also have a regulatory sequence (operator, SD sequence, terminator, etc.) effective for expression of the fusion protein of the present invention in coryneform bacteria at an appropriate position so that it can function.

The promoter used in the present invention is not particularly limited as long as it is a promoter that functions in coryneform bacteria. The term "promoter that functions in coryneform bacteria" means a promoter that has a promoter activity (i.e., gene transcription activity) in coryneform bacteria. Examples of the promoter that functions in coryneform bacteria include those described in the section of "Method of increasing activity of protein" described below.

The signal peptide used in the present invention is not particularly limited as long as it is a signal peptide that functions in coryneform bacteria. The signal peptide may be a signal peptide derived from a coryneform bacterium (for example, derived from the host) or a signal peptide derived from a different species. The term "signal peptide that functions in coryneform bacteria" refers to a peptide that allows coryneform bacteria to secrete a protein such as bbFGF linked with the peptide at the N-terminus thereof. Whether a certain signal peptide functions in coryneform bacteria can be confirmed by, for example, expressing a protein such as bbFGF as a fusion protein with the signal peptide, and confirming whether the protein is secreted.

Examples of the signal peptide include Tat-dependent signal peptides and Sec-dependent signal peptides.

The term "Tat-dependent signal peptide" refers to a signal peptide that is recognized by the Tat system. The "Tat-dependent signal peptide" may specifically be such a peptide that when it is linked to the N-terminus of a protein such as bbFGF, the protein is secreted by the Tat secretion apparatus.

Examples of the Tat-dependent signal peptide include, for example, the signal peptide of the TorA protein (trimethylamine-N-oxidoreductase) of *E. coli,* the signal peptide of the SufI protein (suppressor of ftsI) of *E. coli,* the signal peptide of the PhoD protein (phosphodiesterase) of *Bacillus subtilis,* the signal peptide of the LipA protein (lipoic acid synthase) of *Bacillus subtilis,* and the signal peptide of the IMD protein (isomaltodextranase) of *Arthrobacter globiformis.* The amino acid sequences of these signal peptides are as follows.
TorA signal peptide: MNNNDLFQASRRRFLAQLGGLTVAGMLGPSLLTPRRATA (SEQ ID NO: 18)
SufI signal peptide: MSLSRRQFIQASGIALCAGAVPLKASA (SEQ ID NO: 19)
PhoD signal peptide: MAYDSRFDEWVQKLKEESFQNNTFDRRKFIQGAGKIAGLSLGLTIAQS (SEQ ID NO: 20)
LipA signal peptide: MKFVKRRTTALVTTLMLSVTSLFALQPSAKAAEH (SEQ ID NO: 21)
IMD signal peptide: MMNLSRRTLLTTGSAATLAYALGMAGSAQA (SEQ ID NO: 22)

The Tat-dependent signal peptide has a twin-arginine motif. Examples of the twin-arginine motif include, for example, S/T-R-R-X-F-L-K (SEQ ID NO: 23) and R-R-X-#-# (X represents a naturally occurring amino acid residue, and # represents a hydrophobic amino acid residue).

The "Sec-dependent signal peptide" refers to a signal peptide that is recognized by the Sec system. The "Sec-dependent signal peptide" may specifically be such a peptide that when it is linked to the N-terminus of a protein such as bbFGF, the protein is secreted by the Sec secretion apparatus.

Examples of the Sec-dependent signal peptide include, for example, signal peptides of cell surface layer proteins of coryneform bacteria. The cell surface layer proteins of coryneform bacteria are as described above. Examples of the cell surface layer proteins of coryneform bacteria include PS1 and PS2 (CspB) derived from *C. glutamicum* (Japanese Patent Publication (Kokai) No. Hei 6-502548), and SlpA (CspA) derived from *C*. *stationis* (Japanese Patent Publication (Kokai) No. Hei 10-108675). There are shown the amino acid sequence of the signal peptide of PS1 (PS1 signal peptide) of *C. glutamicum* as SEQ ID NO: 25, the amino acid sequence of the signal peptide of PS2 (CspB) (PS2 signal peptide) of *C*. *glutamicum* as SEQ ID NO: 26, and the amino acid sequence of the signal peptide of SlpA (CspA) (SlpA signal peptide) of *C. stationis* as SEQ ID NO: 27.

The Tat-dependent signal peptide may be a variant of any of the Tat-dependent signal peptides exemplified above as long as it has the twin-arginine motif and the original function is maintained. The Sec-dependent signal peptide may also be a variant of any of the Sec-dependent signal peptides exemplified above as long as it has the twin-arginine motif and the original function is maintained. For variants of the signal peptide and the gene encoding the signal peptide, the description for conserved variants of bbFGF and bbFGF gene described below can be applied. For example, the signal peptide may be a peptide having an amino acid sequence derived from any of the amino acid sequences of the signal peptide exemplified above by substitution, deletion, insertion, and/or addition of one or several amino acids at one or several positions. The term "one or several" used above for the variant of the signal peptide specifically means preferably 1 to 7, more preferably 1 to 5, further preferably 1 to 3, especially preferably 1 or 2. The terms "TorA signal peptide", "SufI signal peptide", "PhoD signal peptide", "LipA signal peptide", "IMD signal peptide", "PS1 signal peptide", "PS2 signal peptide", and "SlpA signal peptide" shall encompass the peptides listed as SEQ ID NOS: 18 to 22 and 25 to 27 and conserved variants thereof.

The expression that "the original function is maintained" used for the Tat-dependent signal peptide may mean that the peptide is recognized by the Tat system, specifically, the peptide has such a function that if it is linked to the N-terminus of a protein such as bbFGF, the protein is secreted by the Tat secretion apparatus. Whether a certain peptide has the function as the Tat-dependent signal peptide can be confirmed by, for example, confirming that secretion amount of a protein to which the peptide is added at the N-terminus is increased by enhancement of the Tat secretion apparatus, or confirming that secretion amount of a protein to which the peptide is added at the N-terminus is decreased by deficiency of the Tat secretion apparatus.

The expression that "the original function is maintained" used for the Sec-dependent signal peptide may mean that the peptide is recognized by the Sec system, specifically, the peptide has such a function that if it is linked to the N-terminus of a protein such as bbFGF, the protein is secreted by the Sec secretion apparatus. Whether a certain peptide has the function as the Sec-dependent signal peptide can be confirmed by, for example, confirming that secretion amount of a protein to which the peptide is added at the N-terminus is increased by enhancement of the Sec secretion apparatus, or confirming that secretion amount of a protein to which the peptide is added at the N-terminus is decreased by deficiency of the Sec secretion apparatus.

Signal peptides are generally cleaved by a signal peptidase when the translation product is secreted out of the bacterial cells. That is, bbFGF finally obtained does not need to have a signal peptide. The gene encoding the signal peptide can be used in its naturally occurring form, or it can be modified to have optimal codons according to the codon usage frequency of the host used.

In the bbFGF expression cassette, a nucleic acid sequence encoding an amino acid sequence containing Gln-Glu-Thr may be inserted between the nucleic acid sequence encoding the signal peptide and the nucleic acid sequence encoding bbFGF (WO2013/062029). The "amino acid sequence containing Gln-Glu-Thr" is also referred to as the "insertion sequence used in the present invention". Examples of the insertion sequence used in the present invention include the amino acid sequences containing Gln-Glu-Thr described in WO2013/062029. The insertion sequence used in the present invention is particularly preferably used in combination with a Sec-dependent signal peptide.

The insertion sequence used in the present invention is preferably a sequence consisting of three or more amino acid residues from the N-terminus of the mature protein of the cell surface layer protein CspB of coryneform bacteria (hereinafter also referred to as "mature CspB" or "CspB mature protein"). The term "sequence consisting of three or more amino acid residues from the N-terminus" refers to the amino acid sequence from the amino acid residue at position 1 of the N-terminus to the amino acid residue at position 3 or any of the following positions.

The cell surface layer protein CspB of coryneform bacteria will be described later. Specific examples of CspB include CspB of *C. glutamicum* ATCC 13869, CspB of 28 strains of *C. glutamicum* described later, and variants thereof. In the amino acid sequence of CspB of *C. glutamicum* ATCC 13869 shown as SEQ ID NO: 11, the amino acid residues at positions 1 to 30 correspond to the signal peptide, and the amino acid residues at positions 31 to 499 correspond to the CspB mature protein. The amino acid sequence of the CspB mature protein of *C*. *glutamicum* ATCC 13869, excluding the 30 amino acid residues of the signal peptide portion, is shown as SEQ ID NO: 28. In the mature CspB of *C. glutamicum* ATCC 13869, the amino acid residues at positions 1 to 3 at the N-terminus correspond to Gln-Glu-Thr.

The insertion sequence used in the present invention is preferably an amino acid sequence from the amino acid residue at position 1 to any one of the amino acid residues at positions 3 to 50 of the mature CspB. The insertion sequence used in the present invention is preferably an amino acid sequence from the amino acid residue at position 1 to any of the amino acid residues at positions 3 to 8, 17, and 50 of the mature CspB. The insertion sequence used in the present invention is particularly preferably an amino acid sequence from the amino acid residue at position 1 to any one of the amino acid residues at positions 4, 6, 17, and 50 of the mature CspB.

The insertion sequence used in the present invention is preferably, for example, an amino acid sequence selected from the group consisting of the amino acid sequences A through H mentioned below.
(A) Gln-Glu-Thr
(B) Gln-Glu-Thr-Xaa1
(C) Gln-Glu-Thr-Xaa1-Xaa2
(D) Gln-Glu-Thr-Xaa1-Xaa2-Xaa3
(E) Amino acid sequence consisting of Gln-Glu-Thr to which amino acid residues at positions 4 to 7 of the mature CspB are added
(F) Amino acid sequence consisting of Gln-Glu-Thr to which amino acid residues at positions 4 to 8 of the mature CspB are added
(G) Amino acid sequence consisting of Gln-Glu-Thr to which amino acid residues at positions 4 to 17 of the mature CspB are added
(H) Amino acid sequence consisting of Gln-Glu-Thr to which amino acid residues at positions 4 to 50 of the mature CspB are added

In the amino acid sequences A to H, Xaa1 is Asn, Gly, Thr, Pro, or Ala, Xaa2 is Pro, Thr, or Val, and Xaa3 is Thr or Tyr. In the amino acid sequences A to H, "Gln-Glu-Thr to which amino acid residues at positions 4 to X of the mature CspB are added" means an amino acid sequence consisting of Gln-Glu-Thr and amino acid residues at positions 4 to X from the N-terminal of the mature CspB added to Thr of Gln-Glu-Thr. The first to third amino acid residues at the N-terminus of the mature CspB are normally Gln-Glu-Thr, in which case "amino acid sequence consisting of Gln-Glu-Thr to which amino acid residues at positions 4 to X of the mature CspB are added" is synonymous with an amino acid sequence consisting of the amino acid residues at positions 1 to X of the mature CspB.

Specifically, the insertion sequence used in the present invention is preferably, for example, an amino acid sequence selected from the group consisting of Gln-Glu-Thr-Asn-Pro-Thr (SEQ ID NO: 32), Gln-Glu-Thr-Gly-Thr-Tyr (SEQ ID NO: 33), Gln-Glu-Thr-Thr-Val-Thr (SEQ ID NO: 34), Gln-Glu-Thr-Pro-Val-Thr (SEQ ID NO: 35), and Gln-Glu-Thr-Ala-Val-Thr (SEQ ID NO: 36).

The term "amino acid residue at position X of the mature CspB" means an amino acid residue corresponding to the amino acid residue at position X in the sequence of SEQ ID NO: 28. Which amino acid residue in the amino acid sequence of any mature CspB is "the amino acid residue corresponding to the amino acid residue at position X in the sequence of SEQ ID NO: 28" can be determined by aligning the amino acid sequence of any such mature CspB with the amino acid sequence of SEQ ID NO: 28.

The term "bovine basic fibroblast growth factor (bbFGF)" may refer to basic fibroblast growth factor derived from bovine. The term "bovine" in reference to bbFGF may refer to organisms belonging to the genus *Bos.* Examples of organisms belonging to the genus *Bos* include *Bos taurus.* The nucleotide sequence of the bbFGF gene and the amino acid sequence of bbFGF can be obtained from, for example, public databases such as NCBI or from technical literature such as patent documents. The nucleotide sequence of the bbFGF gene of *Bos taurus* (modified in consideration of the codon usage frequency of *C*. *glutamicum*) and the amino acid sequence of bbFGF encoded by the gene are shown as SEQ ID NOS: 45 and 46, respectively.

The bbFGF gene may be, for example, a gene having the nucleotide sequence of the bbFGF gene exemplified above (e.g., the nucleotide sequence shown as SEQ ID NO: 45). The bbFGF may be, for example, a protein having the amino acid sequence of bbFGF exemplified above (e.g., the amino acid sequence shown as SEQ ID NO: 46). The expression that "a gene or protein has a nucleotide sequence or an amino acid sequence" may mean that the gene or protein contains such a nucleotide sequence or amino acid sequence, and may also encompass the case where the gene or protein consists of such a nucleotide sequence or amino acid sequence, unless otherwise specified.

The bbFGF gene may be, for example, a variant of a gene having the nucleotide sequence of the bbFGF gene exemplified above. The bbFGF may be, for example, a variant of a protein having the amino acid sequence of bbFGF exemplified above. Such a variant is not particularly limited as long as it has a desired function. Such a variant may be, for example, a conservative variant. The term "conservative variant" refers to a variant in which the original function is maintained. Examples of the variant such as conservative variant include, for example, homologues and artificial modifications of the gene and protein having the nucleotide sequence of the bbFGF gene exemplified above or the amino acid sequence of bbFGF exemplified above. In the case of bbFGF, examples of the homologues include homologues possessed by organisms belonging to the genus *Bos.* The protein identified with the species of origin is not limited to the protein itself found in that species, but encompasses proteins having the amino acid sequence of the protein found in that species and their variants. Such variants may or may not be found in the species. That is, for example, "bbFGF" is not limited to the basic fibroblast growth factor found in an organism belonging to the genus *Bos* itself, but also encompasses proteins having the amino acid sequences of basic fibroblast growth factor found in organisms belonging to the genus *Bos* and variants thereof.

The expression that "the original function is maintained" means that a variant of a gene or protein has a function (e.g., activity or property) that corresponds to the function (e.g., activity or property) of the original gene or protein. For a gene, the expression that "the original function is maintained" means that a variant of the gene encodes a protein in which the original function is maintained. For bbFGF, the expression that "the original function is maintained" may mean that a variant of bbFGF has a function of promoting proliferation of animal cells.

Hereafter, the variants such as conservative variants will be explained with reference to examples.

Homologues of the bbFGF gene and homologues of bbFGF can be easily obtained from public databases by, for example, BLAST search or FASTA search using the nucleotide sequence of the bbFGF gene exemplified above or the amino acid sequence of bbFGF exemplified above as a query sequence. Homologues of the bbFGF gene can also be obtained by, for example, PCR using any of chromosomes of various organisms as the template and oligonucleotides prepared on the basis of the nucleotide sequence of the bbFGF gene exemplified above as primers.

The bbFGF gene may be a gene encoding a protein having an amino acid sequence derived from the amino acid sequence bbFGF exemplified above by substitution, deletion, insertion, and/or addition of one or several amino acids at one or several positions. For example, the encoded protein may have an extended or shortened N- and/or C-terminus. The number meant by the term "one or several" mentioned above means, depending on the positions and types of amino acid residues in the three-dimensional structure of the protein, specifically, for example, 1 to 50, 1 to 40, or 1 to 30, preferably 1 to 20, more preferably 1 to 10, further preferably 1 to 5, especially preferably 1 to 3.

The substitution, deletion, insertion, and/or addition of one or several amino acids mentioned above may be a conservative mutation that maintains the original function of the protein. Typical examples of the conservative mutation include conservative substitutions. Conservative substitutions are such mutations that mutual substitution occurs among Phe, Trp, and Tyr when the substitution site is an aromatic amino acid, among Leu, Ile, and Val when the substitution site is a hydrophobic amino acid, between Gln and Asn when the substitution site is a polar amino acid, among Lys, Arg, and His when the substitution site is a basic amino acid, between Asp and Glu when the substitution site is an acidic amino acid, and between Ser and Thr when the substitution site is an amino acid with hydroxyl group. Specific examples of substitutions considered conservative substitutions include substitution of Ser or Thr for Ala, substitution of Gln, His or Lys for Arg, substitution of Glu, Gln, Lys, His or Asp for Asn, substitution of Asn, Glu or Gln for Asp, substitution of Ser or Ala for Cys, substitution of Asn, Glu, Lys, His, Asp or Arg for Gln, substitution of Gly, Asn, Gln, Lys or Asp for Glu, substitution of Pro for Gly, substitution of Asn, Lys, Gln, Arg or Tyr for His, substitution of Leu, Met, Val or Phe for Ile, substitution of Ile, Met, Val or Phe for Leu, substitution of Asn, Glu, Gln, His or Arg for Lys, substitution of Ile, Leu, Val or Phe for Met, substitution of Trp, Tyr, Met, Ile or Leu for Phe, substitution of Thr or Ala for Ser, substitution of Ser or Ala for Thr, substitution of Phe or Tyr for Trp, substitution of His, Phe or Trp for Tyr, and substitution of Met, Ile or Leu for Val. The aforementioned substitution, deletion, insertion, addition, inversion, etc. of amino acids may be those resulting from a naturally-occurring mutation due to individual difference, difference of species, or the like of the organism from which the gene is derived (mutant or variant).

The bbFGF gene may also be a gene encoding a protein having an amino acid sequence showing an identity of, for example, 50% or higher, 65% or higher, or 80% or higher, preferably 90% or higher, more preferably 95% or higher, further preferably 97% or higher, especially preferably 99% or higher, to the entire sequence of the amino acid sequence of bbFGF exemplified above.

The bbFGF gene may also be a gene, such as DNA, that hybridizes to a probe that can be prepared from the nucleotide sequence of the bbFGF gene exemplified above, for example, a sequence complementary to the entire sequence or a part thereof of the nucleotide sequence of the bbFGF gene exemplified above, under stringent conditions. The term "stringent conditions" refers to conditions under which a so-called specific hybrid is formed, and a non-specific hybrid is not formed. Examples of the stringent conditions include those under which DNAs having a high identity to each other, for example, DNAs having an identity to each other of 50% or higher, 65% or higher, or 80% or higher, preferably 90% or higher, more preferably 95% or higher, further preferably 97% or higher, particularly preferably 99% or higher, hybridize to each other, and DNAs having an identity lower than the above do not hybridize to each other, for example, the washing conditions of usual Southern hybridization, i.e., washing once, preferably 2 or 3 times, at salt concentrations and temperature corresponding to 1 x SSC and 0.1% SDS at 60°C, preferably 0.1 x SSC and 0.1% SDS at 60°C, more preferably 0.1 x SSC and 0.1% SDS at 68°C.

As described above, the probe used for the hybridization mentioned above may be a part of a complementary sequence of the gene. Such a probe can be prepared by PCR using oligonucleotides prepared on the basis of the nucleotide sequence of the bbFGF gene exemplified above as primers and a DNA fragment containing the gene described above as a template. For example, a DNA fragment of about 300 bp in length can be used as the probe. When a DNA fragment having a length of about 300 bp is used as the probe, the washing conditions of the hybridization can be, for example, 50°C, 2 x SSC, and 0.1% SDS.

In addition, since the degeneracy of codons varies from host to host, the bbFGF gene may have replacement of any codon with an equivalent codon. That is, the bbFGF gene may be, for example, a variant of the bbFGF gene exemplified above occurring due to the degeneracy of codons. For example, the bbFGF gene may be modified to have optimal codons according to the codon usage frequency of the host to be used.

The term "identity" between amino acid sequences means an identity between amino acid sequences calculated with blastp using the default settings of Scoring Parameters (Matrix, BLOSUM62; Gap Costs, Existence=11 and Extension=1; and Compositional Adjustments, Conditional compositional score matrix adjustment). The term "identity" between nucleotide sequences means an identity between nucleotide sequences calculated with blastn using the default settings of Scoring Parameters (Match/Mismatch Scores=1, -2; and Gap Costs=Linear).

The above descriptions for variants of bbFGF and bbFGF gene can also be applied to other proteins and nucleotide sequences encoding them.

The bbFGF expression cassette may further contain a nucleic acid sequence encoding an amino acid sequence used for enzymatic cleavage between the nucleic acid sequence encoding an amino acid sequence containing Gln-Glu-Thr and the nucleic acid sequence encoding bbFGF. If such an amino acid sequence used for enzymatic cleavage is inserted into the fusion protein of the present invention, the expressed fusion protein can be enzymatically cleaved to obtain bbFGF.

The amino acid sequence used for enzymatic cleavage is not particularly limited as long as it is a sequence that is recognized and cleaved by an enzyme that hydrolyzes peptide bonds, and any usable sequence may be appropriately selected according to the amino acid sequence of bbFGF. A nucleic acid sequence encoding such an amino acid sequence used for enzymatic cleavage can be appropriately designed on the basis of that amino acid sequence. For example, a nucleic acid sequence encoding an amino acid sequence used for enzymatic cleavage can be designed to have optimal codons depending on the codon usage frequency of the host.

The amino acid sequence used for enzymatic cleavage is preferably a recognition sequence of a protease with high substrate specificity. Specific examples of such an amino acid sequence include, for example, the recognition sequence of Factor Xa protease and the recognition sequence of ProTEV protease. The Factor Xa protease recognizes the amino acid sequence Ile-Glu-Gly-Arg (=IEGR) (SEQ ID NO: 37) in a protein, the ProTEV protease recognizes the amino acid sequence Glu-Asn-Leu-Tyr-Phe-Gln (=ENLYFQ) (SEQ ID NO: 38) in a protein, and they specifically cleave the amino acid sequences on the C-terminal side of the respective recognition sequences.

The N-terminal region of the bbFGF finally obtained may or may not be identical to that of the naturally occurring protein. For example, the N-terminal region of the bbFGF finally obtained may be that of the naturally occurring protein to which or from which one or several amino acids are added or deleted. The number meant by the term "one or several" used above varies depending on the total length and structure of bbFGF, but specifically, preferably 1 to 20, more preferably 1 to 10, further preferably 1 to 5, especially preferably 1 to 3.

The method of introducing the bbFGF expression cassette into a coryneform bacterium is not particularly limited. The term "introducing the bbFGF expression cassette" refers to making the host retain the gene construct. The "introducing the bbFGF expression cassette" is not limited to introducing the components all at once as a pre-constructed gene construct into the host, but also encompasses introducing at least the bbFGF gene into the host so that a gene construct is constructed within the host. In the bacterium of the present invention, the bbFGF expression cassette may be present on a vector that autonomously reproduces outside the chromosome, such as a plasmid, or may be incorporated into the chromosome. The bbFGF expression cassette can be introduced, for example, in the same manner as that of the introduction of gene described in the section of "Method of increasing activity of protein" described later.

The bbFGF expression cassette can be introduced into the host by using, for example, a vector containing the gene construct. For example, the bbFGF expression cassette can be ligated to a vector to construct an expression vector for the gene construct, and the host can be transformed with the expression vector to introduce the gene construct into the host. Further, for example, if the vector is equipped with a promoter that functions in coryneform bacteria, an expression vector of the bbFGF expression cassette can also be constructed by ligating a nucleotide sequence encoding the fusion protein of the present invention downstream of the promoter. The vector is not particularly limited as long as it can autonomously replicate in coryneform bacteria. Vectors usable in coryneform bacteria are as described above.

The bbFGF expression cassette can also be introduced into the host chromosome by using, for example, a transposon such as an artificial transposon. If a transposon is used, the bbFGF expression cassette is introduced into a chromosome by homologous recombination or on the basis of the transposability of the transposon itself. In addition, the bbFGF expression cassette can also be introduced into the host chromosome by an introduction method using homologous recombination. Examples of the introduction method using homologous recombination include, for example, methods using a linear DNA, plasmid containing a temperature-sensitive replication origin, plasmid capable of conjugation transfer, suicide vector not having replication origin that functions within the host, or the like. Alternatively, at least the bbFGF gene may be introduced into a chromosome to construct the bbFGF expression cassette on the chromosome. In such a case, a part or all of the components of the bbFGF expression cassette other than the bbFGF gene may originally exist on the chromosome of the host. Specifically, for example, the bbFGF expression cassette can be constructed on the chromosome by using a promoter sequence and nucleic acid sequence encoding a signal peptide and ligated downstream of the promoter sequence originally existing on the chromosome of the host as they are, and replacing only a gene ligated downstream of the nucleic acid sequence encoding a signal peptide with the bbFGF gene, and thereby the bacterium of the present invention can be constructed. A part of the bbFGF expression cassette such as the bbFGF gene can be introduced into a chromosome in the same manner as that of the introduction of the bbFGF expression cassette into a chromosome.

The bbFGF expression cassette and components thereof (promoter sequence, nucleic acid sequence encoding a signal peptide, nucleic acid sequence encoding bbFGF, etc.) can be obtained by, for example, cloning. Specifically, for example, the bbFGF expression cassette can be obtained by cloning the bbFGF gene from an organism having bbFGF, and conducting modifications such as introduction of a nucleotide sequence encoding a signal peptide and introduction of a promoter sequence. The bbFGF expression cassette and components thereof can also be obtained by chemical synthesis (Gene, 60(1), 115-127 (1987)). The obtained gene construct and the components thereof can be used as they are or after appropriate modifications.

The method of introducing the bbFGF expression cassette into a coryneform bacterium is not particularly limited, and commonly used methods such as protoplast method (Gene, 39, 281-286 (1985)), electroporation method (Bio/Technology, 7) , 1067-1070 (1989)), electric pulse method (Japanese Patent Publication (Kokai) No. Hei 2-207791), etc. can be used.

### 1-3. Other properties

The bacterium of the present invention may have any desired properties as long as it is capable of secretory production of bbFGF. For example, the bacterium of the present invention may have decreased cell surface layer protein activity (WO2013/065869, WO2013/065772, WO2013/118544, and WO2013/062029). The bacterium of the present invention may also be modified to have decreased activity of penicillin-binding protein (WO2013/065869). The bacterium of the present invention may also be modified so that expression of a gene encoding a metallopeptidase is increased (WO2013/065772). The bacterium of the present invention may also be modified to have a mutant ribosomal protein S1 gene (mutant rpsA gene) (WO2013/118544). The bacterium of the present invention may also be modified to have a mutant phoS gene (WO2016/171224). The bacterium of the present invention may also be modified to have decreased activity of the RegX3 protein (WO2018/074578 ). The bacterium of the present invention may also be modified to have decreased activity of the HrrSA system (WO2018/074579). The bacterium of the present invention may also be modified to have increased activity of the Tat secretion apparatus. The bacterium of the present invention may also be modified to have decreased activity of protein O-mannosyltransferase (PMT) (Martina Mahne et al., The *Corynebacterium glutamicum* gene pmt encoding a glycosyltransferase related to eukaryotic protein-O-mannosyltransferases is essential for glycosylation of the resuscitation promoting factor (Rpf2) and other secreted proteins, FEMS Microbiol Lett. 2006 Jun;259(2):226-33). These properties or modifications can be used individually or in appropriate combinations.

### 1-3-1. Introduction of mutant phoS gene

The bacterium of the present invention may be modified to retain a mutant phoS gene. "Retaining a mutant phoS gene" is also referred to as "having a mutant phoS gene" or "having a mutation in the phoS gene". "Retaining a mutant phoS gene" is also referred to as "having a mutant PhoS protein" or "having a mutation in the PhoS protein".

The phoS gene and PhoS protein will be explained below. The phoS gene is a gene encoding the PhoS protein, which is a sensor kinase of the PhoRS system. The PhoRS system is a two-component regulatory system that elicits a response to phosphate deficiency in the environment. The PhoRS system consists of the sensor kinase PhoS encoded by the phoS gene and the response regulator PhoR encoded by the phoR gene.

A PhoS protein having a "specific mutation" is also referred to as a "mutant PhoS protein", and a gene encoding it is also referred to as a "mutant phoS gene". In other words, the "mutant phoS gene" is a phoS gene that has a "specific mutation". Further, a PhoS protein that does not have any "specific mutation" is also referred to as a "wild-type PhoS protein", and a gene encoding it is also referred to as a "wild-type phoS gene". In other words, the "wild-type phoS gene" is a phoS gene that does not have any "specific mutation". The term "wild-type" is used here for convenience to distinguish those from those of "mutant type", and wild-type protein is not limited to naturally occurring proteins as long as it does not have any "specific mutation". The "specific mutation" will be described below.

Examples of the wild-type phoS gene include, for example, the phoS genes of coryneform bacteria. Specific examples of the phoS genes of coryneform bacteria include the phoS genes of *C. glutamicum* YDK010 strain, *C. glutamicum* ATCC 13032 strain, *C. glutamicum* ATCC 14067 strain, *C. callunae, C. crenatum,* and *C. efficiens.* The nucleotide sequence of the phoS gene of *C. glutamicum* YDK010 strain is shown as SEQ ID NO: 1. Furthermore, the amino acid sequences of wild-type PhoS proteins encoded by these phoS genes are shown as SEQ ID NOS: 2 to 7, respectively.

The wild-type phoS gene may be a variant of any of the above-exemplified wild-type phoS genes, as long as it does not have any "specific mutation" and the original function is maintained. Similarly, the wild-type PhoS protein may be a variant of any of the above-exemplified wild-type PhoS proteins, as long as it does not have any "specific mutation" and the original function is maintained. That is, the term "wild-type phoS gene" shall mean not only the above-exemplified wild-type phoS genes, but also conservative variants thereof that do not have any "specific mutation". Similarly, the term "wild-type PhoS protein" shall mean not only the above-exemplified wild-type PhoS proteins, but also conservative variants thereof that do not have any "specific mutation". For variants of the wild-type PhoS protein and wild-type phoS gene, the above description for conservative variants of the bbFGF gene and bbFGF can be applied. For example, the wild-type phoS gene may be a gene encoding a protein having an amino acid sequence derived from any of the amino acid sequences mentioned above by substitution, deletion, insertion, and/or addition of one or several amino acids at one or several positions, as long as it does not have any "specific mutation" and the original function is maintained. Further, the wild-type phoS gene may be, for example, a gene encoding a protein having an amino acid sequence showing an identity of 80% or higher, preferably 90% or higher, more preferably 95% or higher, further preferably 97% or higher, particularly preferably 99% or higher, to the entire sequence of any of the amino acid sequences mentioned above, as long as it does not have any "specific mutation" and the original function is maintained.

The expression that "the original function is maintained" may mean that, in the case of the wild-type PhoS protein, the variant of the protein has the function as the PhoS protein (for example, function of a protein consisting of any of the amino acid sequences shown as SEQ ID NOS: 2 to 7). Furthermore, the expression that "the original function is maintained" may mean that, in the case of a wild-type PhoS protein, the variant of the protein has a function as a sensor kinase of the PhoRS system. That is, the "function as the PhoS protein" may specifically be a function as a sensor kinase of the PhoRS system. Specifically, the "function as a sensor kinase of the PhoRS system" may be a function of conjugating with the PhoR protein, which is a response regulator, to elicit a response to phosphate deficiency in the environment. More specifically, the "function of as a sensor kinase of the PhoRS system" may be a function of being autophosphorylated upon sensing phosphate deficiency in the environment, and activating the PhoR protein through transfer of phosphate group.

Whether or not a variant of the PhoS protein has the function as a sensor kinase of the PhoRS system can be confirmed by, for example, introducing a gene encoding the variant into a strain of a coryneform bacterium deficient in the phoS gene and confirming whether the responsiveness to phosphate deficiency is compensated. Compensation of responsiveness to phosphate deficiency can be detected as, for example, enhanced growth under phosphate deficient conditions or as induction of expression of a gene whose expression is known to be induced under phosphate deficient conditions (J. Bacteriol., 188, 724-732 (2006)). As a phoS gene-deficient strain of coryneform bacteria, for example, the phoS gene-deficient strain of *C. glutamicum* YDK010 or the phoS gene-deficient strain of *C. glutamicum* ATCC 13032 can be used.

The wild-type PhoS protein preferably conserves the histidine residue to be autophosphorylated. That is, the conservative mutation preferably occurs at an amino acid residue other than the histidine residue to be autophosphorylated. The "histidine residue to be autophosphorylated" refers to the histidine residue at position 276 of the wild-type PhoS protein. The wild-type PhoS protein also preferably has, for example, a conserved sequence of any of the wild-type PhoS proteins exemplified above. That is, the conservative mutation preferably occurs, for example, at an amino acid residue that is not conserved in each of the wild-type PhoS proteins exemplified above.

The mutant PhoS protein is a protein having any of the amino acid sequences of the wild-type PhoS proteins described above having a "specific mutation".

That is, in other words, the mutant PhoS protein may be identical to any of the wild-type PhoS proteins and conserved variants thereof exemplified above, except that it has a "specific mutation". Specifically, for example, the mutant PhoS protein may be a protein having any of the amino acid sequences shown as SEQ ID NOS: 2 to 7, except for having a "specific mutation". The mutant PhoS protein may also specifically be, for example, a protein having an amino acid sequence derived from any of the amino acid sequences shown as SEQ ID NOS: 2 to 7 by substitution, deletion, insertion, and/or addition of one or several amino acids. The mutant PhoS protein may also specifically be, for example, a protein having an identity of 80% or higher, preferably 90% or higher, more preferably 95% or higher, still more preferably 97% or higher, particularly preferably 99% or higher, to any of the amino acid sequences shown as SEQ ID NOS: 2 to 7, except for having a "specific mutation".

In other words, the mutant PhoS protein may also be a variant of any of the wild-type PhoS proteins exemplified above that has a "specific mutation" and further contains a conservative mutation at a position other than that of the "specific mutation". Specifically, the mutant PhoS protein may be, for example, a protein having any of the amino acid sequences shown as SEQ ID NOS: 2 to 7 that has a "specific mutation", and further has substitution, deletion, insertion, and/or addition of one or several amino acids at a position other than that of the "specific mutation".

The mutant phoS gene is not particularly limited as long as it encodes such a mutant PhoS protein as described above.

The "specific mutation" of the mutant PhoS protein will be explained below.

The "specific mutation" is not particularly limited as long as it changes the amino acid sequence of the wild-type PhoS protein described above and is effective for secretory production of a heterologous protein such as bbFGF.

The "specific mutation" is preferably a mutation that improves secretory production amount of a heterologous protein such as bbFGF. To "improve secretory production amount of a heterologous protein" means that a coryneform bacterium modified to have the mutant phoS gene (modified strain) is capable of secretory production of a larger amount of the heterologous protein compared with an unmodified strain. The term "unmodified strain" refers to a control strain that does not have a mutation in the phoS gene, i.e., a control strain that does not have any mutant phoS gene, which may be, for example, a wild strain or parent strain. The degree of the larger amount in the expression "secretory production of a larger amount of a heterologous protein compared with an unmodified strain" is not particularly limited as long as the secretory production amount of the heterogenous protein is increased compared with that of the unmodified strain, but it means that, for example, the mutant strain produces and secretes the heterogenous protein in an amount of preferably 1.1 times or more, more preferably 1.2 times or more, further preferably 1.3 times more, further preferably 2 times more, especially preferably 5 times more, of that the unmodified strain in terms of accumulation amount in the medium and/or cell surface layers. The expression that "secretory production of a larger amount of a heterologous protein compared with an unmodified strain" may also mean that when an unconcentrated culture supernatant of the unmodified strain is subjected to SDS-PAGE and staining is performed with CBB, no heterologous protein is detected, but when an unconcentrated culture supernatant of the modified strain is subjected to SDS-PAGE and staining is performed with CBB, the heterologous protein can be detected. For "improving secretory production amount of the heterologous protein", it is not necessary that the secretory production amounts of all heterologous proteins are improved, but it is sufficient if secretory production amount of a heterologous protein set as the target of secretory production is increased. The term "to improve secretory production amount of a heterologous protein" may specifically mean, for example, to improve the secretory production amount of the heterologous protein described in the examples.

Whether a certain mutation is a mutation that improves secretory production amount of a heterogenous protein such as bbFGF can be determined by, for example, preparing a strain that has been modified to have a gene encoding the PhoS protein having the mutation from a strain belonging to coryneform bacteria, quantifying the secretory production amount of the heterogenous protein obtained when the modified strain is cultured in a medium, and comparing it with the secretory production amount of the heterogenous protein obtained when the strain not modified (unmodified strain) is cultured in the medium.

As the change in the amino acid sequence, substitution of an amino acid residue is preferred. That is, the "specific mutation" is preferably substitution of another amino acid residue for an amino acid residue of the wild-type PhoS protein. The amino acid residue for which the substitution occurs due to the "specific mutation" may consist of a single residue, or a combination of two or more residues. The amino acid residue for which the substitution occurs due to the "specific mutation" may preferably be an amino acid residue other than the histidine residue to be autophosphorylated. The amino acid residue for which the substitution occurs due to the "specific mutation" may more preferably be an amino acid residue in the HisKA domain other than the histidine residue to be autophosphorylated. The term "histidine residue to be autophosphorylated" refers to the histidine residue at position 276 of the wild-type PhoS protein. The term "HisKA domain" refers to the region consisting of the amino acid residues at positions 266 to 330 of the wild-type PhoS protein. The amino acid residue for which the substitution occurs due to the "specific mutation" may be especially preferably the tryptophan residue at position 302 (W302) of the wild-type PhoS protein.

For the above mutation, examples of the amino acid residue present after the substitution include K (Lys), R (Arg), H (His), A (Ala), V (Val), L (Leu), I (Ile), G (Gly), S (Ser), T (Thr), P (Pro), F (Phe), W (Trp), Y (Tyr), C (Cys), M (Met), D (Asp), E (Glu), N (Asn), and Q (Gln), which should be other than the original amino acid residue. As the amino acid residue present after the substitution, those that provide improved secretion production amount of a heterologous protein such as bbFGF can be selected.

When W302 is replaced, examples of the amino acid residue present after the substitution include amino acid residues other than those of aromatic amino acids and histidine. Specific examples of "the amino acid residues other than those of aromatic amino acids and histidine" include K (Lys), R (Arg), A (Ala), V (Val), L (Leu), I (Ile), G (Gly), S (Ser), T (Thr), P (Pro), C (Cys), M (Met), D (Asp), E (Glu), N (Asn), and Q (Gln). More specific examples of "the amino acid residues other than those of aromatic amino acids and histidine" include K (Lys), A (Ala), V (Val), S (Ser), C (Cys), M (Met), D (Asp) and N (Asn).

The term "specific mutation" in the phoS gene means a mutation in the nucleotide sequence that causes the above "specific mutation" in the encoded PhoS protein.

The term "amino acid residue at position X of the wild-type PhoS protein" means an amino acid residue corresponding to the amino acid residue at position X in the sequence of SEQ ID NO: 2. For example, "W302" means the amino acid residue corresponding to the tryptophan residue at position 302 in the sequence of SEQ ID NO: 2. The above position of the amino acid residue indicates a relative position, and the absolute position may be changed back and forth due to deletion, insertion, addition, etc. of amino acids. For example, in the wild-type PhoS protein consisting of the amino acid sequence shown as SEQ ID NO: 2, if one amino acid residue is deleted or inserted at a position of the protein on the N-terminus side of the position X, the amino acid residue at the original position X becomes an amino acid residue at position X - 1 or X + 1, counted from the N-terminus, respectively, but these are regarded as the "amino acid residue at position X of the wild-type PhoS protein". Specifically, for example, in the amino acid sequences of the wild-type PhoS protein shown as SEQ ID NOS: 2 to 7, "W302" corresponds to the tryptophan residues at positions 302, 302, 302, 321, 275, and 286, respectively. In the amino acid sequences of the wild-type PhoS protein shown as SEQ ID NOS: 2 to 7, "the histidine residue at position 276 of the wild-type PhoS protein (histidine residue to be autophosphorylated)" corresponds to the histidine residues at positions 276, 276, 276, 295, 249, and 260, respectively. Also in the amino acid sequences of the wild-type PhoS protein shown as SEQ ID NOS: 2 to 7, "the region consisting of the amino acid residues at positions 266 to 330 of the wild-type PhoS protein (HisKA domain)" corresponds to the regions consisting of the amino acid residues at positions 266 to 330, 266 to 330, 266 to 330, 285 to 349, 239 to 303, and 250 to 314, respectively.

The "W302" referred to here is usually a tryptophan residue, but it may not be a tryptophan residue. That is, if the wild-type PhoS protein has an amino acid sequence other than those shown as SEQ ID NOS: 2 to 7, "W302" may not be a tryptophan residue. Therefore, for example, "the mutation that substitutes a cysteine residue for W302" is not limited to a mutation that substitutes a cysteine residue for a tryptophan residue when "W302" is a tryptophan residue, but also encompasses a mutation that substitutes a cysteine residue for K (Lys), R (Arg), H (His), A (Ala), V (Val), L (Leu), I (Ile), G (Gly), S (Ser), T (Thr), P (Pro), F (Phe), Y (Tyr), M (Met), D (Asp), E (Glu), N (Asn), or Q (Gln) as the amino acid residue corresponding to "W302". The same shall apply to other mutations.

In an amino acid sequence of a given PhoS protein, which amino acid residue is "the amino acid residue corresponding to the amino acid residue at position X in the sequence of SEQ ID NO: 2" can be determined by aligning the amino acid sequence of the given PhoS protein with the amino acid sequence of SEQ ID NO: 2. Such alignment can be performed by, for example, using known gene analysis software. Specific examples of such software include DNASIS produced by Hitachi Solutions and GENETYX produced by GENETYX Corporation (Elizabeth C. Tyler et al., Computers and Biomedical Research, 24(1), 72-96, 1991; and Barton GJ et al., Journal of Molecular Biology, 198(2), 327-37. 1987).

The mutant phoS gene can be obtained by, for example, modifying a wild-type phoS gene so that the encoded PhoS protein has the "specific mutation" described above. The wild-type phoS gene as the original to be modified can be obtained by, for example, cloning from an organism having the wild-type phoS gene or chemical synthesis. The mutant phoS gene can also be obtained without using a wild-type phoS gene. For example, the mutant phoS gene can be directly obtained by chemical synthesis. The obtained mutant phoS gene may be further modified and used.

Such genetic modification can be performed by known methods. For example, a site-specific mutagenesis method can be used to introduce a desired mutation at a desired site in DNA. Examples of the site-specific mutagenesis method include methods using PCR (Higuchi, R., 61, in PCR technology, Erlich, H. A. Eds., Stockton press (1989); and Carter, P., Meth. in Enzymol., 154, 382 (1987)), and methods using phage (Kramer, W. and Frits, H. J., Meth. in Enzymol., 154, 350 (1987); and Kunkel, T. A. et al. Meth. in Enzymol., 154, 367 (1987)).

The method of modifying a coryneform bacterium so that the bacterium has the mutant phoS gene will be explained below.

A coryneform bacterium can be modified to have the mutant phoS gene by introducing the mutant phoS gene into the coryneform bacterium. A coryneform bacterium can be modified to have the mutant phoS gene also by introducing the "specific mutation" described above into the phoS gene on the chromosome of the coryneform bacterium. Introduction of a mutation into a gene on the chromosome can be achieved by spontaneous mutation, treatment with a mutagen, or genetic engineering technique.

The method of introducing the mutant phoS gene into a coryneform bacterium is not particularly limited. In the bacterium of the present invention, the mutant phoS gene only needs to be maintained so that it can be expressed under the control of a promoter that functions in the coryneform bacterium. The promoter may be a host-derived promoter or a heterologous promoter. The promoter may be a promoter unique to the phoS gene or a promoter of another gene. In the bacterium of the present invention, the mutant phoS gene may be present on a vector that autonomously replicates outside the chromosome, such as plasmid, or may be integrated into the chromosome. The bacterium of the present invention may have only one copy of the mutant phoS gene, or may have two or more copies thereof. The bacterium of the present invention may have only one type of mutant phoS gene, or may have two or more types of mutant phoS genes. The mutant phoS gene can be introduced, for example, in the same manner as the introduction of a gene in the method of increasing gene expression, which will be described later, or the introduction of the bbFGF expression cassette.

The bacterium of the present invention may or may not have the wild-type phoS gene, but preferably does not have it.

A coryneform bacterium not having the wild-type phoS gene can be obtained by disrupting the wild-type phoS gene on the chromosome. Disruption of the wild-type phoS gene can be performed by known methods. Specifically, for example, the wild-type phoS gene can be disrupted by deleting a part or all of the promoter region and/or coding region of the wild-type phoS gene.

A coryneform bacterium that does not have the wild-type phoS gene and has been modified to have the mutant phoS gene can also be obtained by replacing the wild-type phoS gene on the chromosome with the mutant phoS gene. Examples of the method of such gene replacement include, for example, methods using linear DNA such as the method called "red-driven integration" (Datsenko, K. A., and Wanner, B. L. Proc. Natl. Acad. Sci. USA. 97:6640 -6645 (2000)), red-driven integration method combined with a λ phage-derived excision system (Cho, E. H., Gumport, R. I., Gardner, J. F. J. Bacteriol. 184: 5200-5203 (2002)) (refer to WO2005/010175), method using a plasmid containing a temperature-sensitive replication origin, method using a plasmid capable of conjugation transfer, method using a suicide vector not having a replication origin that functions within the host (U.S. Patent No. 6,303,383, Japanese Patent Publication (Kokai) No. Hei 05-007491), and so forth.

The PhoS protein conjugates with the response regulator, PhoR protein, and functions, i.e., elicits a response to phosphate deficiency in the environment. Therefore, the bacterium of the present invention has the phoR gene so that the mutant PhoS protein functions. The phoR gene is a gene that encodes the PhoR protein, which is the response regulator of the PhoRS system. "Having the phoR gene" is also referred to as "having the PhoR protein". Usually, it is sufficient if the PhoR protein inherently possessed by the bacterium of the present invention conjugates with the mutant PhoS protein and functions. However, an appropriate phoR gene may be introduced into the bacterium of the present invention in addition to or instead of the phoR gene inherently possessed by the bacterium of the present invention. The phoR gene to be introduced is not particularly limited as long as it encodes a PhoR protein that conjugates with the mutant PhoS protein and functions.

Examples of the phoR gene include, for example, the phoR genes of coryneform bacteria. Specific examples of the phoR genes of coryneform bacteria include the phoR genes of *C. glutamicum* YDK010 strain, *C. glutamicum* ATCC 13032 strain, *C. glutamicum* ATCC 14067 strain, *C. callunae, C. crenatum,* and *C. efficiens.* The nucleotide sequence of the phoR gene and the amino acid sequence of the PhoR protein of *C. glutamicum* ATCC 13032 are shown as SEQ ID NOS: 8 and 9, respectively.

The phoR gene may be a variant of any of the phoR genes exemplified above as long as the original function is maintained. Similarly, the PhoR protein may be a variant of any of the PhoR proteins exemplified above as long as the original function is maintained. That is, the term "phoR gene" shall encompass the phoR genes exemplified above as well as conserved variants thereof. Similarly, the term "PhoR protein" shall encompass the PhoR proteins exemplified above as well as conserved variants thereof. The above descriptions for conservative variants of the bbFGF gene and bbFGF can be applied to variants of the PhoR protein and the phoR gene. For example, the phoR gene may be a gene encoding a protein having an amino acid sequence derived from the amino acid sequence mentioned above by substitution, deletion, insertion, and/or addition of one or several amino acids at one or several positions, as long as the original function is maintained. The phoR gene may also be, for example, a gene encoding a protein having an amino acid sequence showing an identity of 80% or higher, preferably 90% or higher, more preferably 95% or higher, further preferably 97% or higher, especially preferably 99% or higher, to the entire sequence of the amino acid sequence mentioned above, as long as the original function is maintained. The expression that "the original function is maintained" may mean that, in the case of the PhoR protein, a variant of the protein has the function as the PhoR protein (e.g., the function of a protein consisting of the amino acid sequence shown as SEQ ID NO: 9). The expression that "the original function is maintained" may also mean that, in the case of the PhoR protein, a variant of the protein has a function as a response regulator of the PhoRS system. That is, the "function as the PhoR protein" may specifically be the function as a response regulator of the PhoRS system. The "function as a response regulator of the PhoRS system" may specifically be a function of conjugating with the PhoS protein, which is a sensor kinase, to elicit a response to phosphate deficiency in the environment. The "function as a response regulator of the PhoRS system" may more specifically be a function of being activated through transfer of phosphate group from the PhoS protein autophosphorylated upon sensing phosphate deficiency in the environment, and regulating expression of a gene that responds to phosphate deficiency in the environment.

Whether or not a variant of the PhoR protein has the function as a response regulator of the PhoRS system can be confirmed by, for example, introducing a gene encoding the variant into a strain of a coryneform bacterium deficient in the phoR gene and confirming whether or not the responsiveness to phosphate deficiency is compensated. Compensation of the responsiveness to phosphate deficiency can be detected as, for example, enhanced growth under phosphate-deficient conditions or induction of expression of a gene whose expression is known to be induced under phosphate-deficient conditions (J. Bacteriol., 188, 724-732 (2006)). As the phoR gene-deficient strain of coryneform bacteria, a phoR gene-deficient strain of *C. glutamicum* YDK010 strain or a phoR gene-deficient strain of *C. glutamicum* ATCC 13032 strain can be used.

### 1-3-2. Decrease in activity of cell surface layer protein

The bacterium of the present invention may be a bacterium having decreased activity of a cell surface layer protein. Specifically, the bacterium of the present invention may be a bacterium having decreased activity of a cell surface layer protein compared with an unmodified strain. The term "decreased activity of a cell surface layer protein" may specifically mean decreased molecular number per cell of the cell surface layer protein. Cell surface layer proteins and genes encoding them will be explained below.

Cell surface layer proteins are proteins that constitute the cell surface layers (S-layer) of bacteria and archaea. Examples of the cell surface layer proteins of coryneform bacteria include PS1, PS2 (CspB), and SlpA (CspA). Specific examples of the cell surface layer proteins of coryneform bacteria include PS1 and PS2 (CspB) of *C. glutamicum* (Japanese Patent Publication (Kohyo) No. Hei 6-502548) and SlpA (CspA) of *C. stationis* (Japanese Patent Publication (Kokai) No. Hei 10-108675). Among these, it is preferable to decrease the activity of the PS2 protein.

The nucleotide sequence of the cspB gene of *C. glutamicum* ATCC 13869 and the amino acid sequence of the PS2 protein (CspB protein) encoded by the gene are shown as SEQ ID NOS: 10 and 11, respectively.

Further, for example, amino acid sequences of CspB homologues have been reported for 28 strains of *C. glutamicum* (J Biotechnol., 112, 177-193 (2004)). These 28 strains of *C. glutamicum* and the GenBank accession numbers in the NCBI database for the cspB gene homologues thereof are listed below (GenBank accession numbers are shown in the parentheses).
*C. glutamicum* ATCC 13058 (AY524990)
*C. glutamicum* ATCC 13744 (AY524991)
*C. glutamicum* ATCC 13745 (AY524992)
*C. glutamicum* ATCC 14017 (AY524993)
*C. glutamicum* ATCC 14020 (AY525009)
*C. glutamicum* ATCC 14067 (AY524994)
*C. glutamicum* ATCC 14068 (AY525010)
*C. glutamicum* ATCC 14747 (AY525011)
*C. glutamicum* ATCC 14751 (AY524995)
*C. glutamicum* ATCC 14752 (AY524996)
*C. glutamicum* ATCC 14915 (AY524997)
*C. glutamicum* ATCC 15243 (AY524998)
*C. glutamicum* ATCC 15354 (AY524999)
*C. glutamicum* ATCC 17965 (AY525000)
*C. glutamicum* ATCC 17966 (AY525001)
*C. glutamicum* ATCC 19223 (AY525002)
*C. glutamicum* ATCC 19240 (AY525012)
*C. glutamicum* ATCC 21341 (AY525003)
*C. glutamicum* ATCC 21645 (AY525004)
*C. glutamicum* ATCC 31808 (AY525013)
*C. glutamicum* ATCC 31830 (AY525007)
*C. glutamicum* ATCC 31832 (AY525008)
*C. glutamicum* LP-6 (AY525014)
*C. glutamicum* DSM20137 (AY525015)
*C. glutamicum* DSM20598 (AY525016)
*C. glutamicum* DSM46307 (AY525017)
*C. glutamicum* 22220 (AY525005)
*C. glutamicum* 22243 (AY525006)

Because differences may exist in the nucleotide sequences of genes encoding the cell surface layer proteins depending on differences in the species or strain to which the coryneform bacteria belong, the gene encoding the cell surface layer protein may be a variant of any of the genes encoding the cell surface layer proteins exemplified above, as long as the original function is maintained. Similarly, the cell surface layer protein may be a variant of any of the cell surface layer proteins exemplified above as long as the original function is maintained. That is, for example, the term "cspB gene" shall encompass the cspB genes exemplified above as well as conserved variants thereof. Similarly, the term "CspB protein" shall include the CspB proteins exemplified above and conserved variants thereof. For variants of cell surface layer proteins and genes encoding them, the above description for conserved variants of bbFGF genes and bbFGF can be applied. For example, the gene encoding the cell surface layer protein may be a gene encoding a protein having an amino acid sequence derived from any of the aforementioned amino acid sequences by substitution, deletion, insertion, and/or addition of one or several amino acids at one or several positions. Further, for example, the gene encoding the cell surface layer protein may be a gene encoding a protein having an amino acid sequence showing an identity of 80% or higher, preferably 90% or higher, more preferably 95% or higher, further preferably 97% or higher, particularly preferably 99% or higher, to the entire sequence of any of the amino acid sequences mentioned above, as long as the original function is maintained. The expression that "the original function is maintained" may mean that, for example, in the case of the cell surface layer protein, the protein has a property of increasing secretory production amount of a heterologous protein such as bbFGF compared with an unmodified strain when the activity thereof is decreased in a coryneform bacterium.

The term "property of increasing secretory production amount of a heterologous protein compared with an unmodified strain when the activity thereof is decreased in a coryneform bacterium" refers to a property of conferring on the coryneform bacterium an ability of secretory production of the heterologous protein in a greater amount compared with an unmodified strain when the activity of the protein is decreased in the coryneform bacterium. The term "unmodified strain" refers to a control strain in which the activity of the cell surface layer protein is not decreased, which may be, for example, a wild strain or parent strain. The degree of the "secretory production of the heterologous protein in a greater amount compared with an unmodified strain" is not particularly limited as long as the secretory production amount of the heterologous protein is increased compared with the unmodified strain, but it may mean, for example, secretory production of the heterologous protein in an amount of, preferably 1.1 times or more, more preferably 1.2 times or more, further preferably 1.3 times or more, especially preferably 2 times or more, of that of the unmodified strain in terms of accumulation amount in the medium and/or cell surface layers. The term "secretory production of the heterologous protein in a greater amount compared with an unmodified strain" may also mean that when unconcentrated culture supernatant of the unmodified strain is subjected to SDS-PAGE and staining is performed with CBB, no heterologous protein is detected, but when unconcentrated culture supernatant of the modified strain is subjected to SDS-PAGE and staining is performed with CBB, the heterologous protein can be detected.

Whether a certain protein has the property of increasing secretory production amount of a heterologous protein such as bbFGF when the activity thereof is decreased in a coryneform bacterium compared with an unmodified strain can be confirmed by preparing a strain modified to decrease the activity of the protein from a strain belonging to coryneform bacteria, quantifying the secretory production amount of the heterologous protein obtained when the modified strain is cultured in a medium, and comparing the amount with the secretory production amount of the heterologous protein obtained when the strain is cultured in the medium before it is modified (unmodified strain).

The "decrease in activity of a cell surface layer protein" may be due to that the bacterium of the present invention is modified so that the activity of the cell surface layer protein is decreased, or that the activity of the cell surface layer protein is originally decreased in the bacterium of the present invention. The case where "the activity of the cell surface layer protein is originally decreased in the coryneform bacterium" encompasses the case where the coryneform bacterium originally does not have the cell surface layer protein. That is, examples of the coryneform bacterium in which the activity of the cell surface layer protein is decreased includes, for example, a coryneform bacterium that does not originally have the cell surface layer protein. Examples of such "a coryneform bacterium that does not originally have the cell surface layer protein" include, for example, a coryneform bacterium that does not originally have any gene encoding the cell surface layer protein. The expression that "a coryneform bacterium does not originally have the cell surface layer protein" may mean that the coryneform bacterium does not originally have one or more types of proteins selected from the cell surface layer proteins found in other strains of the species to which that coryneform bacterium belongs. For example, an expression that *"C. glutamicum* does not originally have a cell surface layer protein" may means that the *C. glutamicum* strain does not originally have one or more types of proteins selected from cell surface layer proteins found in other *C. glutamicum* strains, that is, such as PS1 and/or PS2 (CspB). Examples of such a coryneform bacterium that does not originally have a cell surface layer protein include, for example, *C. glutamicum* ATCC 13032 that does not originally have the cspB gene.

### 1-3-3. Protein secretion system

The bacterium of the present invention has a protein secretion system. The bacterium of the present invention may inherently have a protein secretion system. The protein secretion system is not particularly limited as long as it can secrete a protein such as bbFGF. Examples of the protein secretion system include the Sec system (Sec secretion apparatus) and Tat system (Tat secretion apparatus). The bacterium of the present invention may be modified so that the activity of the protein secretion system (e.g., Tat secretion system) is increased. Specifically, the bacterium of the present invention may be modified so that the activity of the protein secretion system (e.g., Tat secretion apparatus) is increased compared with that of an unmodified strain. The activity of the Tat secretion system can be increased by increasing expression of one or more types of genes selected from the genes encoding the Tat secretion apparatus proteins. That is, the bacterium of the present invention may be modified so that expression of one or more types of genes selected from the genes encoding the Tat secretion apparatus proteins is increased. Increase of the activity of the Tat secretion apparatus is particularly suitable for the secretory production of bbFGF using a Tat-dependent signal peptide. Techniques for increasing expression of the genes encoding the Tat secretion apparatus proteins are described in Japanese Patent No. 4730302.

Examples of the genes encoding the Tat secretion apparatus proteins include the tatA gene, tatB gene, tatC gene, and tatE gene.

Specific examples of the genes encoding the Tat secretion apparatus proteins include the tatA gene, tatB gene, and tatC gene of *C. glutamicum* ATCC 13032. The tatA gene, tatB gene, and tatC gene of *C. glutamicum* ATCC 13032 correspond to complementary sequences of the sequences of the positions 1571065 to1571382, 1167110 to 1167580, and 1569929 to 1570873 in the genome sequence registered in the NCBI database as GenBank accession NC_003450 (VERSION NC_003450.3 GI:58036263), respectively. The TatA protein, TatB protein, and TatC protein of *C. glutamicum* ATCC 13032 are registered as GenBank accession NP_600707 (version NP_600707.1 GI:19552705, locus_tag="NCgl1434"), GenBank accession NP_600350 (version NP _600350.1 GI:19552348, locus_tag="NCgl1077"), and GenBank accession NP_600706 (version NP_600706.1 GI:19552704, locus_tag="NCgl1433"), respectively. The nucleotide sequences of the tatA gene, tatB gene, and tatC gene and the amino acid sequences of the TatA protein, TatB protein, and TatC protein of *C. glutamicum* ATCC 13032 are shown as SEQ ID NOS: 12 to 17, respectively.

Specific examples of the genes encoding the Tat secretion apparatus proteins also include the tatA gene, tatB gene, tatC gene, and tatE gene of *E. coli.* The tatA gene, tatB gene, tatC gene, and tatE gene of *E. coli* K-12 MG1655 correspond to the sequences at the positions 4019968 to 4020237, 4020241 to 4020756, 4020759 to 4021535, and 658170 to 658373 in the genome sequence registered in the NCBI database as GenBank accession NC_000913 (VERSION NC_000913.2 GI:49175990), respectively. The TatA protein, TatB protein, TatC protein, and TatE protein of *E. coli* K-12 MG1655 are registered as GenBank accession NP_418280 (version NP_418280.4 GI:90111653, locus_tag="b3836"), GenBank accession YP_026270 (version YP _026270.1 GI:49176428, locus_tag="b3838"), GenBank accession NP_418282 (version NP _418282.1 GI:16131687, locus_tag="b3839"), and GenBank accession NP _415160 (version NP _415160.1 GI: 16128610, locus _tag="b0627"), respectively.

The genes encoding the Tat secretion apparatus proteins may be a variant of any of the genes encoding the Tat secretion apparatus proteins exemplified above, as long as the original function is maintained. Similarly, the Tat secretion apparatus proteins may be a variant of the Tat secretion apparatus proteins exemplified above, as long as the original function is maintained. That is, for example, the terms "tatA gene," "tatB gene," "tatC gene," and "tatE gene" shall encompass the tatA gene, tatB gene, tatC gene, and tatE gene exemplified above as well as conserved variants thereof, respectively. Similarly, the terms "TatA protein," "TatB protein," "TatC protein," and "TatE protein" shall encompass the TatA protein, TatB protein, TatC protein, and TatE protein exemplified above as well as conserved variants thereof, respectively. For variants of the Tat secretion apparatus proteins and the genes encoding the Tat secretion apparatus proteins, the above description for conserved variants of the bbFGF gene and bbFGF can be applied. For example, the genes encoding the Tat secretion apparatus proteins may be a gene encoding a protein having an amino acid sequence derived from any of the aforementioned amino acid sequences by substitution, deletion, insertion, and/or addition of one or several amino acids at one or several positions, as long as the original function is maintained. The genes encoding the Tat secretion apparatus proteins may also be, for example, a gene encoding a protein having an amino acid sequence showing an identity of 80% or higher, preferably 90% or higher, more preferably 95% or higher, further preferably 97% or higher, particularly preferably 99% or higher, to the entire sequence of any of the amino acid sequences mentioned above, as long as the original function is maintained. The expression that "the original function is maintained" may mean, in the case of the Tat secretion apparatus, it has the function of extracellularly secreting a protein having a Tat-dependent signal peptide added at the N-terminus.

Increase of the activity of the Tat secretion apparatus can be confirmed by, for example, confirming increase of the secretory production amount of a protein to which the Tat-dependent signal peptide has been added at the N-terminus. The secretory production amount of the protein having the Tat-dependent signal peptide added at the N-terminus thereof may be increased by, for example, 1.5 times or more, 2 times or more, or 3 times or more, compared with an unmodified strain.

### 1-3-4. Decrease in PMT activity

The bacterium of the present invention may be a bacterium with decreased activity of protein O-maltosyltransferase (PMT). Specifically, the bacterium of the present invention may be a bacterium of which activity of PMT is decreased compared with an unmodified strain. The expression "activity of PMT is decreased" may mean that, especially, the number of PMT molecules per cell is decreased. The PMT and the gene encoding it will be explained below.

The term "protein O-mannosyltransferase (PMT)" may refer to a protein that has the activity to catalyze the O-maltosylation reaction of a protein to be produced and secreted. This activity is also referred to as the "PMT activity". The gene encoding PMT is also referred to as the "PMT gene".

The nucleotide sequences of the PMT gene and the amino acid sequences of PMT of coryneform bacteria can be obtained from, for example, public databases such as NCBI or from technical literature such as patent documents. The PMT gene of *C. glutamicum* ATCC 13869 (also referred to as "pmt1 gene"), and the amino acid sequence of the PMT encoded by the gene (also referred to as "PMT1") are shown as SEQ ID NOS: 39 and 40, respectively.

Because differences may exist in the sequence of the PMT gene depending on the species or strain to which the coryneform bacterium belongs, the PMT gene may be a variant of the PMT gene exemplified above, as long as the original function is maintained. Similarly, PMT may be a variant of the PMT exemplified above, as long as the original function is maintained. That is, the term "PMT gene" shall encompass the PMT gene exemplified above as well as conserved variants thereof. Similarly, the term "PMT" shall encompass PMT exemplified above as well as conserved variants thereof. For variants of PMT and the gene encoding it, the above description for conservative variants of the bbFGF gene and bbFGF can be applied. For example, the PMT gene may be a gene encoding a protein having an amino acid sequence derived from the amino acid sequences mentioned above by substitution, deletion, insertion, and/or addition of one or several amino acids at one or several positions, as long as the original function is maintained. Further, the PMT gene may also be, for example, a gene encoding a protein having an amino acid sequence showing an identity of 80% or higher, preferably 90% or higher, more preferably 95% or higher, further preferably 97% or higher, particularly preferably 99% or higher, to the entire sequence of the amino acid sequence mentioned above, as long as the original function is maintained. The expression that "the original function is maintained" may mean that, for example, in the case of PMT, the variant has the PMT activity.

Whether a certain protein has the PMT activity can be confirmed by preparing a strain modified to reduce the activity of the protein from a strain belonging to coryneform bacteria, and confirming that the degree of O-glycosylation of a protein produced and secreted when the modified strain is cultured in a medium is decreased compared with the degree of O-glycosylation of the protein produced and secreted when the strain is cultured in the medium before the modification (unmodified strain).

### 1-4. Method of increasing activity of protein

Techniques for increasing activity of a protein (including techniques for increasing gene expression) will be explained below.

The expression that "activity of a protein is increased" means that the activity of the protein is increased compared with an unmodified strain. Specifically, the expression that "activity of a protein is increased" means that the activity of the protein per cell is increased compared with an unmodified strain. The "unmodified strain" referred to here means a control strain that has not been modified to decrease the activity of the target protein. Examples of the unmodified strain include a wild-type strain and parent strain. Specific examples of the unmodified strain include type strains of respective bacterial species. Specific examples of the unmodified strain also include the strains exemplified in the explanation of the coryneform bacteria. That is, in one embodiment, activity of a protein may be increased compared with a type strain (i.e., a type strain of the species to which the bacterium of the present invention belongs). In another embodiment, activity of a protein may be increased compared with the *C. glutamicum* ATCC 13869 strain. In another embodiment, activity of a protein may be increased compared with *C. glutamicum* ATCC 13032 strain. In another embodiment, activity of a protein may be increased compared with *C. glutamicum* AJ12036 (FERM BP-734). In another embodiment, activity of a protein may be increased compared with *C. glutamicum* YDK010 strain. The expression that "activity of a protein is increased" is also expressed as "activity of a protein is enhanced". More specifically, the expression that "activity of a protein is increased" may mean that the number of molecules of the protein per cell is increased and/or the function per molecule of the protein is increased compared with an unmodified strain. That is, the term "activity" used in the expression that "activity of a protein is increased" is not limited to catalytic activity of the protein, but may also mean transcription amount (mRNA amount) or translation amount (protein amount) of the gene encoding the protein. The "number of molecules of the protein per cell" may mean average value of numbers of molecules of the protein per cell. The expression that "activity of a protein is increased" encompasses not only that the activity of the protein is increased in a strain that originally has the activity of the target protein, but also that the activity of the protein is imparted to a strain that does not originally have the activity of the target protein. In addition, as long as the activity of the protein is eventually increased, the activity of the target protein originally possessed by the host may be decreased or eliminated, and then activity of a preferred target protein may be imparted.

The degree of increase in activity of a protein is not particularly limited as long as the activity of the protein is increased compared with an unmodified strain. Activity of a protein may be increased by, for example, 1.5 times or more, 2 times or more, or 3 times or more compared with an unmodified strain. If the unmodified strain does not have the activity of the target protein, it is sufficient that the protein is produced as a result of introducing a gene encoding the protein, for example, the protein is produced to such an extent that the activity thereof can be measured.

Such modification to increase activity of a protein can be achieved by, for example, increasing expression of the gene encoding the protein. The expression that "expression of a gene is increased" means that expression of the gene is increased compared with an unmodified strain, such as a wild strain or parent strain. The expression that "expression of a gene is increased" specifically means that expression amount of the gene per cell is increased compared with the unmodified strain. The term "expression amount of a gene per cell" may mean average value of expression amount of the gene per cell. The expression that "expression of a gene is increased" may mean that, more specifically, transcription amount of the gene (mRNA amount) and/or translation amount (protein amount) of the gene is increased. The expression that "expression of a gene is increased" is also expressed as "expression of a gene is enhanced". Expression of the gene may be increased by, for example, 1.5 times or more, 2 times or more, or 3 times or more, compared with that of the unmodified strain. In addition, the term "to increase expression of a gene" means not only to increase expression of the gene in a strain in which the target gene is originally expressed, but also to make a strain in which the target gene is not originally expressed express the gene. That is, the term "to increase expression of a gene" may mean to, for example, introduce the target gene into a strain that does not carry the gene and make the strain express the gene.

Increase of gene expression can be achieved by, for example, increasing copy number of the gene.

Increase of copy number of a gene can be achieved by introducing the gene into the host chromosome. Introduction of a gene into a chromosome can be achieved by using, for example, homologous recombination (Miller, J. H. Experiments in Molecular Genetics, 1972, Cold Spring Harbor Laboratory). Examples of the method of gene transfer using homologous recombination include, for example, the method using linear DNA such as the red-driven integration method (Datsenko, K. A., and Wanner, B. L. Proc. Natl. Acad. Sci. USA. 97:6640-6645 (2000)), method using a plasmid containing a temperature-sensitive replication origin, method using a plasmid capable of conjugation transfer, method using a suicide vector without a replication origin that functions within the host, and transduction method using phage. Specifically, a target gene can be introduced into a host chromosome by transforming the host with a recombinant DNA containing the gene, and allowing homologous recombination of the gene with the target site on the host chromosome. The structure of the recombinant DNA used for the homologous recombination is not particularly limited as long as homologous recombination occurs in a desired manner. For example, by transforming the host with a linear DNA containing the target gene and having sequences homologous to upstream and downstream sequences of the target site on the chromosome at both ends, and allowing homologous recombination upstream and downstream of the target site, respectively, the target site can be replaced with the gene. The recombinant DNA used for the homologous recombination may contain a marker gene for selecting transformants. Only one copy of the gene may be introduced, or two or more copies may be introduced. For example, by performing homologous recombination targeting a sequence that is present on the chromosome in a large number of copies, a large number of copies of the gene can be introduced into the chromosome. Examples of such a sequence present on the chromosome in a large number of copies include repetitive DNA sequences (repetitive DNA) and inverted repeats present at both ends of a transposon. Such homologous recombination may also be performed by targeting an appropriate sequence on the chromosome, such as a gene that is not required for the production of the target substance. A gene can also be randomly introduced into the chromosome by using a transposon or Mini-Mu (Japanese Patent Publication (Kokai) No. Hei 2-109985, US 5,882,888, and EP 805867B1). Such methods for modification of a chromosome using homologous recombination can be utilized for not only introduction of a target gene, but also for any modification of a chromosome, such as modification of an expression regulatory sequence.

Introduction of a target gene into a chromosome can be confirmed by Southern hybridization using a probe having a sequence complementary to all or a part of the gene, PCR using primers prepared on the basis of the sequence of the gene, or the like.

Copy number of a gene can also be increased by introducing a vector containing the gene into the host. For example, a DNA fragment containing the target gene can be ligated with a vector that functions in the host to construct an expression vector for the gene, and the host can be transformed with the expression vector to increase the copy number of the gene. The DNA fragment containing the target gene can be obtained by, for example, PCR using genomic DNA of a microorganism having the target gene as a template. As the vector, a vector capable of autonomous replication in the host cell can be used. The vector is preferably a multi-copy vector. For selection of transformants, the vector preferably has a marker such as an antibiotic resistance gene. The vector may also contain a promoter or terminator for expression of the inserted gene. The vector may be, for example, a vector derived from a bacterial plasmid, vector derived from a yeast plasmid, vector derived from a bacteriophage, cosmid, phagemid, or the like. Specific examples of vectors capable of autonomous replication in coryneform bacteria include, for example, pHM1519 (Agric. Biol. Chem., 48, 2901-2903 (1984)); pAM330 (Agric. Biol. Chem., 48, 2901-2903 (1984)); plasmids as improved versions of the foregoing ones having a drug resistance gene; pCRY30 (Japanese Patent Publication (Kokai) No. Hei 3-210184); pCRY21, pCRY2KE, pCRY2KX, pCRY31, pCRY3KE, and pCRY3KX (Japanese Patent Publication (Kokai) No. Hei 2-72876, and U.S. Patent No. 5,185,262); pCRY2 and pCRY3 (Japanese Patent Publication (Kokai) No. Hei 1-191686); pAJ655, pAJ611, and pAJ1844 (Japanese Patent Publication (Kokai) No. Sho 58-192900); pCG1 (Japanese Patent Publication (Kokai) No. Sho 57-134500); pCG2 (Japanese Patent Publication (Kokai) No. Sho 58-35197); pCG4 and pCG11 (Japanese Patent Publication (Kokai) No. Sho 57-183799); pVK7 (Japanese Patent Publication (Kokai) No. Hei 10-215883); pVK9 (US2006-0141588); pVC7 (Japanese Patent Publication (Kokai) No. Hei 9-070291); pVS7 (WO2013/069634); pPK4 (Japanese Patent Publication (Kokai) No. Hei 9-322774); and pPK5 (WO2018/074579). Specific examples of vectors capable of autonomous replication in coryneform bacteria also include, for example, pVC7N (Shuhei Hashiro et al., High copy number mutants derived from Corynebacterium glutamicum cryptic plasmid pAM330 and copy number control, J Biosci Bioeng, 2019 May;127(5):529-538), pVC7H1, pVC7H2, pVC7H3, pVC7H4, pVC7H5, pVC7 H6, and pVC7H7 (WO2018/179834), which are variants of pVC7. Specific examples of vectors capable of autonomous replication in coryneform bacteria also include, for example, pPK4H1, pPK4H2, pPK4H3, pPK4H4, pPK4H5, and pPK4H6 (WO2018/179834), which are variants of pPK4. Examples of the vector include, among others, pVC-based vectors and pPK-based vectors. Examples of the pVC-based vectors include pVC7 and variants thereof, these vectors in which their antibiotic resistance genes are replaced with another antibiotic resistance gene, and vectors having a nucleotide sequence identity to any one of the foregoing vectors of 90% or higher, 95% or higher, 97% or higher, or 99% or higher. Examples of the pPK-based vectors include pPK4, pPK5, variants thereof, these vectors in which their antibiotic resistance genes are replaced with another antibiotic resistance gene, and vectors having a nucleotide sequence identity to any one of the foregoing vectors of 90% or higher, 95% or higher, 97% or higher, or 99% or higher. Particularly preferred examples of the vector include pVC7, pVC7N, pPK4, and pPK5.

When a gene is introduced, it is sufficient that the gene is retained in the host so that it can be expressed. Specifically, the gene may be retained so that it can be expressed under the control of a promoter that functions in the host. The promoter is not particularly limited so long as it functions in the host. The term "promoter that functions in the host" refers to a promoter that has a promoter activity in the host. The promoter may be a promoter derived from the host or a promoter of heterologous origin. The promoter may be a promoter unique to the gene to be introduced, or a promoter of another gene. The promoter may also be inducible or constitutive for gene expression.

Examples of promoters that can be used in coryneform bacteria include promoters of genes of the glycolytic system, pentose phosphate pathway, TCA cycle, amino acid biosynthesis systems, and cell surface layer proteins. Specific examples of promoters of amino acid biosynthesis system genes include, for example, promoters of glutamate dehydrogenase gene of the glutamate biosynthesis system, glutamine synthetase gene of the glutamine synthesis system, aspartokinase gene of the lysine biosynthesis system, homoserine dehydrogenase gene of the threonine biosynthesis system, acetohydroxy acid synthase gene of the isoleucine and valine biosynthesis systems, 2-isopropyl malate synthase gene of the leucine biosynthesis system, glutamate kinase gene of the proline and arginine biosynthesis systems, phosphoribosyl-ATP pyrophosphorylase gene of the histidine biosynthesis system, deoxyarabinohepturonic acid phosphate (DAHP) synthase gene of the biosynthesis systems of aromatic amino acids such as tryptophan, tyrosine, and phenylalanine, phosphoribosyl pyrophosphate (PRPP) amidotransferase gene, inosinate dehydrogenase gene, and guanylate synthase gene of the biosynthesis systems of nucleic acids such as inosinic acid and guanylic acid. Examples of promoters that can be used in coryneform bacteria also include the stronger promoters described below.

A terminator for transcription termination can be placed downstream of the gene. Such a terminator is not particularly limited as long as it can function in the host. The terminator can be a terminator derived from the host or a terminator of heterologous origin. The terminator may be a terminator unique to the gene to be introduced, or may be a terminator of another gene. Specific examples of the terminator include, for example, the terminator of bacteriophage BFK20.

Vectors, promoters, and terminators that can be used in various microorganisms are described in detail in, for example, "Microbiology Basic Course 8: Gene Engineering, Kyoritsu Shuppan, 1987", and they can be used.

When two or more genes are introduced, it is sufficient that each gene is retained in the host so that it can be expressed. For example, such two or more genes all may be carried on a single expression vector, or all may be carried on a chromosome. Such two or more genes may also be separately carried on multiple expression vectors, or may be separately carried on a single or multiple expression vectors and a chromosome. An operon may also be constituted with such two or more genes, and introduced.

The gene to be introduced is not particularly limited as long as it encodes a protein that functions in the host. The gene to be introduced may be a gene derived from the host or a gene of heterologous origin. For example, the gene to be introduced can be obtained by PCR using primers designed on the basis of the nucleotide sequence of the gene and the genomic DNA of the organism carrying the gene or a plasmid carrying the gene as a template. The gene to be introduced may also be, for example, entirely synthesized on the basis of the nucleotide sequence of the gene (Gene, 60(1), 115-127 (1987)). The obtained gene can be used as it is or after modified as appropriate. That is, a variant of the gene can be obtained by modifying the gene. The gene can be modified by known methods. For example, a site-directed mutagenesis method can be used to introduce a desired mutation at a desired site in DNA. That is, for example, the coding region of the gene can be modified so that the encoded protein contains substitution, deletion, insertion, and/or addition of an amino acid residue at a specific site by the site-specific mutagenesis method. Examples of the site-specific mutagenesis methods include methods using PCR (Higuchi, R., 61, in PCR technology, Erlich, H. A. Eds., Stockton press (1989); and Carter, P., Meth. in Enzymol. 154, 382 (1987)) or methods using phage (Kramer, W. and Frits, H. J., Meth. in Enzymol., 154, 350 (1987); and Kunkel, T. A., et al. Meth. in Enzymol., 154, 367 (1987)). Alternatively, a variant of the gene may be entirely synthesized.

When a protein functions as a complex consisting of multiple subunits, all of those multiple subunits may be modified, or only a part of them may be modified, as long as the protein activity is eventually increased. That is, for example, when activity of such a protein is increased by increasing expression of a gene, expression of all of the multiple genes encoding those subunits may be enhanced, or expression of only a part of them may be enhanced. Usually, it is preferable to enhance expression of all of the multiple genes encoding those subunits. Further, the subunits constituting the complex may be derived from one type of organism or two or more types of different organisms, as long as the complex has the function of the target protein. That is, for example, genes derived from the same organism and encoding multiple subunits may be introduced into the host, or such genes derived from different organisms may be introduced into the host.

Increase in expression of a gene can also be achieved by improving transcription efficiency of the gene. Increase in expression of a gene can also be achieved by improving translation efficiency of the gene. Improvement of gene transcription efficiency and translation efficiency can be achieved by, for example, modifying an expression regulatory sequence. The term "expression regulatory sequence" is a generic term for sites that affect expression of a gene. Examples of the expression regulatory sequence include promoter, Shine-Dalgarno (SD) sequence (also referred to as ribosome-binding site (RBS)), and spacer region between RBS and the start codon. The expression regulatory sequence can be determined by using a promoter search vector or gene analysis software such as GENETYX. Such an expression regulatory sequence can be modified by, for example, the method using a temperature-sensitive vector or the red-driven integration method (WO2005/010175).

Improvement of gene transcription efficiency can be achieved by, for example, replacing the promoter of the gene on the chromosome with a stronger promoter. The term "stronger promoter" means a promoter that provides improved gene transcription compared with that provided by the originally present wild-type promoter. Examples of stronger promoters that can be used in coryneform bacteria include P54-6 promoter, of which design was artificially changed (Appl. Microbiol. Biotechnol., 53, 674-679 (2000)), pta, aceA, aceB, adh, and amyE promoters, which can be induced with acetic acid, ethanol, pyruvic acid, etc., and other strong promoters such as cspB, SOD, tuf (EF-Tu) promoters (Journal of Biotechnology 104 (2003) 311-323; and Appl Environ Microbiol. 2005 Dec;. 71(12):8587-96), lac promoter, tac promoter, trc promoter, F1 promoter, T7 promoter, T5 promoter, T3 promoter, and SP6 promoter. As a stronger promoter, a highly active type of a conventionally available promoter may also be obtained by using any of various reporter genes. For example, by making the -35 and -10 regions within the promoter region closer to the consensus sequence, the promoter activity can be increased (WO00/18935). Examples of such a highly active type promoter include various tac-like promoters (Katashkina JI et al. Russian Federation Patent Application No. 2006134574). Methods for evaluating strength of promoters and examples of strong promoters are described in the paper of Goldstein et al. (Prokaryotic promoters in biotechnology. Biotechnol. Annu. Rev., 1, 105-128 (1995)), etc.

Improvement of gene translation efficiency can be achieved by, for example, replacing the Shine-Dalgarno (SD) sequence (also referred to as ribosome-binding site (RBS)) of a gene on a chromosome with a stronger SD sequence. The term "stronger SD sequence" means an SD sequence that improves translation of mRNA compared with the originally present wild-type SD sequence. Example of such a stronger SD sequence include, for example, RBS of the gene 10 derived from bacteriophage T7 (Olins P. O. et al, Gene, 1988, 73, 227-235). Furthermore, it is known that substitution, insertion or deletion of several nucleotides in the spacer region between RBS and the start codon, especially in the sequence immediately upstream of the start codon (5'-UTR), greatly affects mRNA stability and translation efficiency, and translation efficiency of a gene can also be improved by modifying them.

Improvement of gene translation efficiency can also be achieved by, for example, modifying codons. For example, translation efficiency of a gene can be improved by replacing a rare codon in the gene with a synonymous codon that is used more frequently. That is, the gene to be introduced may be modified to have codons that are optimized, for example, according to the frequency of codon usage in the host to be used. Substitution of codon can be performed by, for example, the site-specific mutagenesis method. Alternatively, a gene fragment including codon substitution may be synthesized in its entirety. Codon usage frequencies in various organisms are disclosed in the "Codon Usage Database" (http://www.kazusa.or.jp/codon; Nakamura, Y. et al, Nucl. Acids Res., 28, 292 (2000)).

Increase in expression of a gene can also be achieved by amplifying a regulator that increases gene expression or by deleting or weakening a regulator that decreases gene expression.

Such methods for increasing expression of a gene as mentioned above may be used individually or in any appropriate combinations.

Such a modification that activity of a protein is increased can also be achieved by, for example, enhancing specific activity of the protein. A protein with enhanced specific activity can be obtained by, for example, searching various organisms. A highly active type of a protein may also be obtained by introducing a mutation into an existing protein. The mutation to be introduced may be, for example, such a mutation that substitution, deletion, insertion, and/or addition of one or several amino acids is introduced into the protein at one or several positions. The introduction of the mutation can be performed by, for example, such a site-specific mutagenesis method as described above. The introduction of the mutation may also be performed by, for example, a mutation treatment. Examples of the mutation treatment include X-ray irradiation, UV irradiation, and treatment with a mutation agent such as N-methyl-N'-nitro-N-nitrosoguanidine (MNNG), ethyl methanesulfonate (EMS), and methyl methanesulfonate (MMS). DNA may also be treated directly with hydroxylamine in vitro to induce random mutations. The enhancement of specific activity may be used alone or in any combination with any of such methods for enhancing gene expression as mentioned above.

The transformation method is not particularly limited and conventionally known methods can be used. For example, the method of increasing DNA permeability of receptor cells by treating the cells with calcium chloride, as reported for *Escherichia coli* K-12 (Mandel, M. and Higa, A., J. Mol. Biol. 1970, 53, 159-162), or method of preparing competent cells from cells in the growth phase and introducing DNA into them, as reported for *Bacillus zubutillis* (Duncan, C. H., Wilson, G. A., and Young, F. E., 1977. Gene 1: 153-167) can be used. Alternatively, the method of making cells of DNA-receiving bacterium be in a state of protoplast or spheroplast that readily incorporates recombinant DNA, and introducing a recombinant DNA into the cells of DNA-receiving bacterium, as is known for *Bacillus zubutilis,* actinomycetes, and yeasts (Chang, S. and Choen, S. N., 1979. Mol. Gen. Genet. 168:111-115; Bibb, M. J., Ward, J. M. and Hopwood, O. A. 1978. Nature 274: 398-400; and Hinnen, A., Hicks, J. B. and Fink, G. R. 1978. Proc. Natl. Acad. Sci. USA 75:1929-1933) can also be applied. Alternatively, the electric pulse method (Japanese Patent Publication (Kokai) No. Hei 2-207791), as reported for coryneform bacteria, can also be used.

Increase of activity of a protein can be confirmed by measuring the activity of the protein.

Increase of an activity of a protein can also be confirmed by confirming that expression of the gene encoding the protein has increased. Increase of expression of a gene can be confirmed by confirming that transcription amount of the gene has increased or by confirming that the amount of the protein expressed from the gene has increased.

Increase of transcription amount of a gene can be confirmed by comparing amount of mRNA transcribed from the gene with that of an unmodified strain such as wild-type strain or parent strain. Examples of method of evaluating mRNA amount include Northern hybridization, RT-PCR, microarray method, RNA-seq, and so forth (Sambrook, J., et al., Molecular Cloning: A Laboratory Manual/Third Edition, Cold Spring Harbor Laboratory Press, Cold Spring Harbor (USA), 2001). The amount of mRNA (e.g., number of molecules per cell) may be increased by, e.g., 1.5 times or more, 2 times or more, or 3 times or more, compared with that of an unmodified strain.

Increase of amount of a protein can be confirmed by Western blotting using antibodies (Sambrook, J., et al., Molecular Cloning: A Laboratory Manual/Third Edition, Cold Spring Harbor Laboratory Press, Cold Spring Harbor (USA), 2001). The amount of the protein (e.g., number of molecules per cell) may be increased by, for example, 1.5 times or more, 2 times or more, or 3 times or more, compared with that of an unmodified strain.

Such methods for increasing activity of proteins as mentioned above can be used for enhancing activity of any protein or expression of any gene.

### 1-5. Method of decreasing activity of protein

A method of decreasing activity of a protein will be explained below. The method of decreasing activity of a protein described below can also be used for disruption of the wild-type PhoS protein.

The expression that "activity of a protein is decreased" means that activity of the protein is decreased compared with an unmodified strain. The expression that "activity of a protein is decreased" specifically means that the activity of the protein per cell is decreased compared with an unmodified strain. The "unmodified strain" referred to here means a control strain that has not been modified so that the activity of the target protein is decreased. Examples of the unmodified strain include wild-type strain and parent strain. Specific examples of the unmodified strain include a type strain of each bacterial species. Specific examples of the unmodified strain also include the strains exemplified in the explanation of the coryneform bacteria. That is, in one embodiment, the activity of the protein may be decreased compared with a type strain (i.e., the type strain of the species to which the bacterium of the present invention belongs). In another embodiment, the activity of the protein may be decreased compared with *C. glutamicum* ATCC 13032. In another embodiment, the activity of the protein may be decreased compared with *C. glutamicum* ATCC 13869. In another embodiment, the activity of the protein may be decreased compared with *C. glutamicum* AJ12036 (FERM BP-734). In another embodiment, the activity of the protein may be decreased compared with *C. glutamicum* YDK010 strain. The state that "activity of a protein is decreased" encompasses the state that the activity of the protein is completely lost. The expression that "activity of a protein is decreased" may more specifically mean that number of molecules per cell of the protein is decreased and/or function per molecule of the protein is decreased compared with an unmodified strain. That is, "activity" referred to in the expression that "activity of a protein is decreased" is not limited to the catalytic activity of the protein, but may also mean transcription amount (mRNA amount) or translation amount (protein amount) of the gene encoding the protein. The term "number of molecules per cell of the protein" may mean average value of number of molecules of the protein per cell. The state that "number of molecules per cell of the protein is decreased" encompasses a state that the protein is not present at all. The state that "function per molecule of a protein is decreased" encompasses a state that the function per molecule of the protein is completely lost. The degree of decrease in the activity of the protein is not particularly limited as long as the activity of the protein is decreased compared with an unmodified strain. The activity of the protein may be decreased to, for example, 50% or less, 20% or less, 10% or less, 5% or less, or 0% of that of the unmodified strain.

Such a modification that activity of a protein is decreased can be achieved by, for example, decreasing expression of the gene encoding the protein. The expression that "expression of a gene is decreased" means that expression of the gene is decreased compared with an unmodified strain. The expression that "expression of a gene is decreased" specifically means that the expression amount per cell of the gene is decreased compared with an unmodified strain. The "expression amount per cell of the gene" may mean average value of expression amount per cell of the gene. The expression that "expression of a gene is decreased" may more specifically mean that transcription amount (mRNA amount) of the gene is decreased and/or translation amount (protein amount) of the gene is decreased. The state that "expression of a gene is decreased" encompasses a state that the gene is not expressed at all. The expression that "expression of a gene is decreased" is also expressed as "expression of a gene is attenuated". Expression of the gene may be decreased to, for example, 50% or less, 20% or less, 10% or less, 5% or less, or 0% of that of the unmodified strain.

Expression of the gene may be decreased due to, for example, decrease in transcription efficiency, decrease in translation efficiency, or a combination thereof. Decrease in gene expression can be achieved by, for example, modifying an expression regulatory sequence of the gene. The term "expression regulatory sequence" is a generic term for sites that affect gene expression, such as promoter, Shine-Dargano (SD) sequence (also called ribosome-binding sites (RBS)), and spacer region between RBS and the start codon. The expression regulatory sequence can be determined by using, for example, a promoter search vector or gene analysis software such as GENETYX. When an expression regulatory sequence is modified, the expression regulatory sequence is preferably modified for one or more nucleotides, more preferably two or more nucleotides, especially preferably three or more nucleotides. Decrease in gene transcription efficiency can be achieved by, for example, replacing the promoter of the gene on the chromosome with a weaker promoter. The term "weaker promoter" means a promoter that attenuate transcription of the gene compared with the originally existing wild-type promoter. Examples of such a weaker promoter include, for example, an inducible promoter. That is, an inducible promoter can function as a weaker promoter under non-inducible conditions (e.g., in the absence of an inducer). Further, a part or all of the region of the expression regulatory sequence may be deleted (made deficient). Decrease in expression of a gene can also be achieved by, for example, manipulating a factor involved in the regulation of the expression. Examples of such a factor involved in the regulation of the expression include low molecules (inducer, inhibitor, etc.), proteins (transcription factor, etc.), nucleic acids (siRNA, etc.) and so forth involved in transcription and translation regulation. Decrease in expression of a gene can also be achieved by, for example, introducing a mutation into the coding region of the gene so that expression of the gene is decreased. For example, expression of a gene can be decreased by replacing a codon in the coding region of the gene with a synonymous codon that is used at a lower frequency in the host. Expression of a gene itself may also be decreased by, for example, such disruption of the gene as described below.

Such a modification of a protein that the activity thereof is decreased can also be achieved by, for example, disrupting the gene that encodes the protein. The term "disruption of a gene" means such a modification that the gene no longer produces a protein that normally functions. The state that "a protein that normally functions is not produced" encompasses a state that no protein is produced from the gene at all, and a state that a protein of which function per molecule is decreased or lost (e.g., activity or property) is produced from the gene.

Disruption of a gene can be achieved by, for example, deleting the gene on a chromosome (making the gene deficient). The term "deletion of a gene" refers to deletion of a part or all of the coding region of the gene. Further, the entire gene including the sequences present upstream and downstream of the coding region of the gene on the chromosome may be deleted. The sequences present upstream and downstream of the coding region of the gene may include, for example, an expression regulatory sequence of the gene. The region to be deleted may be any region, such as an N-terminus region (region encoding the N-terminus side of the protein), internal region, or C-terminus region (region encoding the C-terminus side of the protein), as long as decrease in the activity of the protein can be achieved. Usually, the longer the region to be deleted, the more reliably the gene can be inactivated. The region to be deleted may be, for example, a region having a length of 10% or more, 20% or more, 30% or more, 40% or more, 50% or more, 60% or more, 70% or more, 80% or more, 90% or more, or 95% or more of the total length of the coding region of the gene. It is preferred that reading frames of the sequences existing upstream and downstream of the region to be deleted should not match each other. Mismatched reading frames can result in a frame shift downstream of the region to be deleted.

Disruption of a gene can also be achieved by, for example, introducing amino acid substitution (missense mutation), termination codon (nonsense mutation), or addition or deletion of one or two nucleotides (frameshift mutation) in the coding region of the gene on the chromosome (Journal of Biological Chemistry 272:8611-8617 (1997), Proceedings of the National Academy of Sciences, USA 95 5511-5515 (1998), and Journal of Biological Chemistry 26 116, 20833-20839 (1991)).

Disruption of a gene can also be achieved by, for example, inserting another nucleotide sequence into the coding region of the gene on the chromosome. The insertion site can be in any region of the gene, but the longer the nucleotide sequence to be inserted, the more reliably the gene can be inactivated. It is preferred that reading frames of the sequences existing upstream and downstream of the insertion site should not match each other. Mismatched reading frames can result in a frame shift downstream of the insertion site. The other nucleotide sequence is not particularly limited as long as it decreases or eliminates the activity of the encoded protein, but examples include, for example, a marker gene such as antibiotic resistance gene and a gene useful in the production of a target substance.

The disruption of the gene may be carried out, in particular, so that the amino acid sequence of the encoded protein is deleted (made deficient). In other words, such a modification that the activity of the protein is decreased can be achieved by, for example, deleting the amino acid sequence (a part or total region of the amino acid sequence) of the protein, specifically, by modifying the gene to encode a protein in which the amino acid sequence (a part or total region of the amino acid sequence) is deleted. The term "deletion of an amino acid sequence of a protein" refers to deletion of a part or all of the amino acid sequence of the protein. In addition, "deletion of an amino acid sequence of a protein" also means that the original amino acid sequence is no longer present in the protein, and encompasses such a modification that the original amino acid sequence is changed to another amino acid sequence. That is, for example, a region changed to another amino acid sequence by frame shift may be regarded as a deleted region. Deletion of an amino acid sequence in a protein typically shortens the total length of the protein, but may not change the total length of the protein, or even extend the total length of the protein. For example, deletion of a part or all of coding region of a gene can result in deletion of a region encoded by the deleted region in the amino acid sequence of the encoded protein. Further, for example, by introducing a termination codon into a coding region of a gene, a region encoded by a region downstream of the site at which the termination codon is introduced can be deleted in the amino acid sequence of the encoded protein. Furthermore, for example, by introducing frame shift into a coding region of a gene, the region encoded by the frame shift site can be deleted. For the position and length of the region to be deleted in the deletion of an amino acid sequence, the description for the position and length of the region to be deleted in the deletion of a gene can be applied.

Such a modification of a gene on a chromosome as described above can be achieved by, for example, preparing a disrupted gene through such a modification of the original gene that it does not produce a protein that normally functions, transforming a host with a recombinant DNA containing the disrupted gene, and allowing homologous recombination between the disrupted gene and the wild-type gene on the chromosome to substitute the disrupted gene for the wild-type gene on the chromosome. In this process, if a marker gene selected according to nutritional requirements and other traits of the host is contained in the recombinant DNA, operations can be facilitated. Examples of the disrupted gene include a gene of which a part or all of the coding region of the gene is deleted, gene introduced with a missense mutation, gene introduced with a nonsense mutation, gene introduced with a frame shift mutation, or gene introduced with an insertion sequence such as transposon or marker gene. The protein encoded by the disrupted gene, if produced, will have a three-dimensional structure different from that of the wild-type protein and will have decreased function or lose the function. The structure of the recombinant DNA used for the homologous recombination is not particularly limited as long as homologous recombination occurs in a desired manner. For example, a host can be transformed with a linear DNA containing the disrupted gene and having upstream and downstream sequences of the wild-type gene on the chromosome at both ends to cause homologous recombination upstream and downstream of the wild-type gene, respectively, and thereby the wild-type gene can be replaced with the disrupted gene. Such gene disruption based on gene substitution utilizing homologous recombination has already been established, and there are methods using linear DNA such as the method called "red-driven integration" (Datsenko, K. A., and Wanner, B. L. Proc. Natl. Acad. Sci. USA. 97:6640 -6645 (2000)), and the red-driven integration method combined with a λ phage-derived excision system (Cho, E. H., Gumport, R. I., Gardner, J. F. J. Bacteriol. 184: 5200-5203 (2002)) (refer to WO2005/010175), method using a plasmid containing a temperature-sensitive replication origin, method using a plasmid capable of conjugation transfer, method using a suicide vector without a replication origin that functions within the host (U.S. Patent No. 6,303,383, Japanese Patent Publication (Kokai) No. Hei 05-007491), and so forth. Use of such methods for modifying a chromosome using homologous recombination as mentioned above is not limited to disruption of a target gene, but they can also be used for any modification of chromosomes, such as modification of an expression regulatory sequence.

Such a modification that activity of a protein is decreased may be performed by, for example, a mutation treatment. Examples of the mutation treatment include X-ray irradiation, UV irradiation, and treatment with a mutation agent such as N-methyl-N'-nitro-N-nitrosoguanidine (MNNG), ethyl methanesulfonate (EMS), and methyl methanesulfonate (MMS).

Such methods for decreasing activity of a protein may be used individually or in appropriate combinations.

Decrease in activity of a protein can be confirmed by measuring the activity of the protein.

Decrease in activity of a protein can also be confirmed by confirming that expression of the gene encoding the protein has decreased. Decrease in expression of a gene can be confirmed by confirming that transcription amount of the gene has decreased or by confirming that amount of the protein expressed from the gene has decreased.

Decrease in transcription amount of a gene can be confirmed by comparing amount of mRNA transcribed from the gene with that of an unmodified strain. Examples of methods for evaluating mRNA amount include Northern hybridization, RT-PCR, microarray method, RNA-seq, and so forth (Sambrook, J., et al., Molecular Cloning: A Laboratory Manual/Third Edition, Cold Spring Harbor Laboratory Press, Cold Spring Harbor (USA), 2001). The amount of mRNA may be decreased to, for example, 50% or less, 20% or less, 10% or less, 5% or less, or 0% of that of the unmodified strain.

Decrease in protein amount can be confirmed by performing SDS-PAGE and confirming intensity of the band of the protein separated. Decrease in protein amount can also be confirmed by Western blotting using antibodies (Sambrook, J., et al., Molecular Cloning: A Laboratory Manual/Third Edition, Cold Spring Harbor Laboratory Press, Cold Spring Harbor (USA), 2001). The amount of protein (e.g., number of molecules per cell) may be decreased to, for example, 50% or less, 20% or less, 10% or less, 5% or less, or 0% of that of an unmodified strain.

Disruption of a gene can be confirmed by determining nucleotide sequence, restriction enzyme map, full length of a part or all of the gene or the like, depending on the means used for the disruption.

The methods for decreasing activity of a protein mentioned above can be used for decreasing activity of any protein or expression of any gene.

### 1-6. Production of bbFGF

If the bacterium of the present invention is cultured, bbFGF is produced and secreted, and thus a culture product containing bbFGF can be obtained.

The medium used for the culture is not particularly limited as long as the bacterium of the present invention can grow and bbFGF is produced. For example, ordinary culture media used for culturing bacteria such as coryneform bacteria can be used as the medium. Specific examples of the medium include, for example, ordinary culture media containing, a carbon source, a nitrogen source, inorganic ions, etc. Organic trace nutrients such as vitamins and amino acids can also be added to the medium as needed. The culture conditions are not particularly limited as long as the bacterium of the present invention can grow and bbFGF is produced. The culture can be carried out, for example, under the usual conditions used to culture bacteria such as coryneform bacteria. The types and concentrations of the medium ingredients and the culture may be determined as appropriate according to various conditions such as the type of the coryneform bacterium.

As the carbon source, for example, carbohydrates such as glucose and sucrose, organic acids such as acetic acid, alcohols, or other suitable carbon sources can be used. As the nitrogen source, for example, ammonia gas, aqueous ammonia, ammonium salts, or other suitable nitrogen sources can be used. As the inorganic ions, for example, calcium ions, magnesium ions, phosphate ions, potassium ions, iron ions and so forth can be appropriately used as needed. The culture can be performed as appropriate, for example, under aerobic conditions at pH 5.0 to 8.5 and 15 to 37°C for about 1 to 7 days. In addition, the culture conditions for L-amino acid production by coryneform bacteria and other conditions described for the production of proteins using Sec- and Tat-dependent signal peptides can be used (see WO01/23591 and WO2005/103278). If an inducible promoter is used for expression of a gene such as the bbFGF gene, the expression of the gene may be induced as required. If the bacterium of the present invention is cultured under such conditions as described above, bbFGF is produced and secreted, and thus a medium containing bbFGF can be obtained.

Secretory production of bbFGF can be confirmed by performing SDS-PAGE for the culture supernatant and/or a fraction containing the cell surface layers as a sample, and confirming molecular weights of proteins of separated bands. Secretory production of bbFGF can also be confirmed by Western blotting using an antibody and the culture supernatant and/or a fraction containing cell surface layers as a sample (Molecular Cloning (Cold Spring Harbor Laboratory Press, Cold Spring Harbor (USA), 2001)). Secretory production of bbFGF can also be confirmed by detecting the N-terminal amino acid sequence of the protein produced and secreted using a protein sequencer. Secretory production of bbFGF can also be confirmed by determining the mass of the produced and secreted protein using a mass spectrometer. If the bbFGF is an enzyme or has a certain measurable biological activity, secretory production of bbFGF can be confirmed by measuring the biological activity of bbFGF in a sample of the culture supernatant and/or fraction containing cell surface layers.

A culture product containing bbFGF (specifically, culture product containing bbFGF produced and secreted) may be used in the production of cultured meat as an active ingredient as it is or after being subjected to a treatment such as removal of cells, reduction, purification, solvent substitution, and drying as appropriate. These treatments may be performed individually or in appropriate combinations. For example, at least removal of cells may be performed. The term "removal of cells" may mean removal of cells of the bacterium of the present invention from the culture product containing bbFGF. The removal of cells can be performed by, for example, solid-liquid separation means such as spontaneous sedimentation, centrifugation, and filtration. Filtration can be performed by using, for example, a filter such as a PVDF membrane. The term "reduction" may refer to reduction of bbFGF, and may specifically mean placing a bbFGF-containing fraction (e.g., culture product, culture supernatant, or purified bbFGF solution) under a reductive condition. Reduction can be performed by, for example, addition of a reducing agent such as DTT. The term "purification" may mean purification of bbFGF, and may specifically mean purification of bbFGF from a bbFGF-containing fraction (e.g., medium or culture supernatant). The term "purification of bbFGF" may mean separating bbFGF and ingredients other than bbFGF. The ingredients other than bbFGF are also referred to as "contaminants". Examples of contaminants include those contained in a fraction containing bbFGF (e.g., culture supernatant). The removal of cells shall not be considered as the "purification of bbFGF". In other words, cells of the bacterium of the present invention shall not be "contaminants" in the purification of bbFGF. The purification can be carried out by means of separation and purification means for proteins, for example, salting out, ethanol precipitation, ultrafiltration, gel filtration chromatography, ion exchange chromatography, reverse phase chromatography, affinity chromatography, etc. In one embodiment, any purification of bbFGF may not be performed. The expression that "purification of bbFGF is not performed" may be used interchangeably with the expression that "bbFGF is not purified". The expression that "purification of bbFGF is not performed" may mean that, for example, bbFGF is used as the active ingredient in a state that 70% (w/w) or more, 80% (w/w) or more, 90% (w/w) or more, 95% (w/w) or more, 97% (w/w) or more, or 99% (w/w) or more of the contaminants remain (i.e., coexist with bbFGF). In one embodiment, in particular, purification of bbFGF with chromatography may not be performed. Not performing purification of bbFGF (in particular, purification with chromatography) may, for example, reduce the production cost of bbFGF, thereby reducing the production cost of cultured meat. bbFGF produced by using a coryneform bacterium as an expression host can be suitably used for animal cell culture (specifically, production of cultured meat) without purification to a high purity with chromatography, etc. The term "solvent substitution" may mean replacement of the liquid portion constituting the bbFGF-containing fraction (e.g., medium, culture supernatant, or purified bbFGF solution). A buffer solution such as PBS may be used for the solvent substitution. Such a buffer solution may contain a reducing agent such as DTT. The term "drying" may mean drying of bbFGF, specifically drying a fraction containing bbFGF (e.g., medium, culture supernatant, or purified bbFGF solution). The drying can be performed by, for example, drying means such as lyophilization or spray drying. If bbFGF is secreted on the bacterial cell surface layers, bbFGF can be solubilized by solubilizing means such as increasing salt concentration or using a surfactant. A fraction containing solubilized bbFGF may be used as the active ingredient in the production of cultured meat as it is or after being subjected to a treatment such as removal of cells, reduction, purification, solvent substitution, and drying, as appropriate, as in the case of a culture product containing bbFGF.

The form of the active ingredient is not particularly limited. The active ingredient may be in any form, for example, in the form of powder, flake, paste, liquid, etc. For example, a fraction containing bbFGF may be dried and powdered, and then the powder may be used as the active ingredient.

### 2. Method of the present invention

The method of the present invention is a method of producing cultured meat, comprising a step of culturing animal cells in the presence of the active ingredient (i.e., bbFGF produced by using a coryneform bacterium as an expression host). This step is also referred to as the "step of culturing animal cells". The step of culturing animal cells may specifically be a step of culturing animal cells in the presence of the active ingredient to form cultured meat.

The animal cells are not particularly limited as long as they can form cultured meat when they are cultured. The cultured meat may consist mainly of muscle tissues. Therefore, the expression that "animal cells can form cultured meat when they are cultured" may mean that, for example, the animal cells can differentiate and form muscle tissues when they are cultured, or specifically that the animal cells can differentiate into myotube cells and form muscle tissues when they are cultured. Examples of the animal cells include stem cells such as mesenchymal stem cells, embryonic stem cells (ES cells), induced pluripotent stem cells (iPS cells), and myoblasts. Examples of the animals include animals for meat. Examples of the animals for meat include cattle, sheep, goat, pig, and rabbit. Examples of the animals for meat include, in particular, cattle and pig.

The culture of the animal cells is carried out in the presence of the active ingredient. The expression that "the culture of the animal cells is carried out in the presence of the active ingredient" may mean that, for example, the medium used for culturing the animal cells contains the active ingredient. The expression that "the culture of the animal cells is carried out in the presence of the active ingredient" may mean that, for example, the active ingredient is supplied to the culture system during culturing the animal cells.

The medium composition and culture conditions are not particularly limited as long as cultured meat is formed by the cultured cells, except that the culture is performed in the presence of the active ingredient. The medium composition and culture conditions can be appropriately set according to various conditions, such as, for example, the type of the animal cells. Except that the culture is performed in the presence of the active ingredient, the culture can be performed by, for example, using a usual medium and usual conditions used for the formation of muscle tissues by differentiation of animal cells (e.g., production of cultured meat) as they are or with modifications as appropriate.

The culture can be performed by using, for example, a liquid medium. The culture can be performed as batch culture, fed-batch culture, continuous culture, or a combination thereof. Examples of the continuous culture include perfusion culture and chemostat culture. The medium used at the start of the culture is also referred to as "starting medium" or "basal medium". The medium supplied to the culture system (e.g., starting medium) in the fed-batch culture is also referred to as "feed medium". The medium supplied to the culture system (e.g., starting medium) in the continuous culture (not limited to the perfusion culture) is also referred to as "perfusion medium". Supplying feed medium or perfusion medium to the culture system in the fed-batch culture or continuous culture is also referred to simply as "medium supply". The medium supply may be performed throughout the entire culture period or during only a part of the culture period. The medium supply may be performed continuously or intermittently. During the culture (especially continuous culture such as perfusion culture), the medium may be withdrawn. The medium may be withdrawn throughout the entire culture period or during only a part of the culture period. The medium may be withdrawn continuously or intermittently. The medium may or may not be withdrawn and supplied simultaneously. The culture may be performed three-dimensionally by using, for example, a scaffold material that matches the shape of the cultured meat to be formed.

The medium used for the culture can be selected, for example, independently for the basal medium, feed medium, and perfusion medium.

The medium used for the culture may be a commercially available medium or a medium appropriately prepared.

Examples of the medium used for the culture include media containing ingredients essential for culturing animal cells (e.g., carbon source, nitrogen source, inorganic salts, etc.).

Specific examples of the medium used for the culture include Dulbecco's Modified Eagle's Medium (DMEM), Ham's F12 medium (Ham's Nutrient Mixture F12), DMEM/F12 medium, McCoy's 5A medium, Minimum Essential Medium (MEM), Eagle's Minimum Essential Medium (EMEM), alpha Modified Eagle's Minimum Essential Medium (αMEM), Roswell Park Memorial Institute (RPMI) 1640 medium, Iscove's Modified Dulbecco's Medium (IMDM), MCDB 131 medium, William's Medium E, and Fischer's Medium.

Further, specific examples of the medium used for the culture (which may be especially, for example, a medium used for culturing stem cells (especially pluripotent stem cells)) include STEMPRO^{®} hESC SFM medium (Life Technologies), mTeSR1 medium (STEMCELL Technologies), TeSR2 medium (STEMCELL Technologies), TeSR-E8 medium (STEMCELL Technologies), Essential 8 medium (Life Technologies), HEScGRO (trademark) Serum-Free Medium for hES cells (Millipore), PluriSTEM (trademark) Human ES/iPS Medium (EMD Millipore), NutriStem^{®} hESC XF medium (Biological Industries Israel Beit-Haemek), NutriStem^{®} XF/FF Culture Medium (Stemgent), AF NutriStem^{®} hESC XF Medium (Biological Industries Israel Beit-Haemek), S-medium (DS Pharma Biomedical Corporation), StemFit^{®} AK03N medium (Ajinomoto Co., Ltd.), hESF9 medium, hESF-FX medium, CDM medium, DEF-CS 500 Xeno-Free 3D Spheroid Culture Medium (Cellartis), StemFlex medium (Thermo Fisher Scientific), etc.

Examples of other commercially available media include media for animal cell culture such as CELLiST Basal Media BASAL3, BASAL4P, BASAL10 (Ajinomoto Co., Ltd.), Opti-MEM (Thermo Fisher Scientific), RPMI 1640 (Thermo Fisher Scientific), CD293 (Thermo Fisher Scientific), CHO-S-SFMII (Thermo Fisher Scientific), CHO-SF (Sigma-Aldrich), EX-CELL CD CHO (Sigma-Aldrich), EX-CELLTM302 (Sigma-Aldrich), IS CHO-CD (Irvine Scientific), and IS CHO-CDXP (Irvine Scientific).

Such media as exemplified above may be used for the culture, for example, after adding the active ingredient.

The medium may contain a variety of medium ingredients. Examples of the medium ingredients include carbon sources, amino acid sources, peptides, proteins, vitamins, fatty acids, lipids, inorganic ingredients, pH-buffering agents, growth factors, cytokines, hormones, cell adhesion factors, extracellular matrix components, serum, antibiotics, and gene expression inducers. Any of these medium ingredients can be essential or effective for, for example, survival or proliferation of animal cells. Any of these medium ingredients may be, for example, contained in such media as exemplified above beforehand, or may be added to such media as exemplified above.

Examples of the carbon sources include sugars such as glucose, fructose, sucrose, and maltose.

Examples of the amino acid sources include amino acids. Peptides and proteins both can also be examples of the amino acid sources. Examples of the amino acids include glycine, alanine, valine, leucine, isoleucine, cysteine, methionine, phenylalanine, tyrosine, tryptophan, histidine, lysine, arginine, serine, threonine, aspartic acid, glutamic acid, asparagine, glutamine, proline, and ornithine. The amino acids may be, for example, L-amino acids.

Examples of the peptides include dipeptides and tripeptides. Specific examples of the peptides include glycylglycylglycine and soy peptides. The description for amino acids can be applied to amino acids constituting peptides.

Examples of the proteins include albumin and transferrin.

Examples of the vitamins include vitamin A, vitamin B1, vitamin B2, vitamin B3, vitamin B5, vitamin B6, vitamin B7, vitamin B9, vitamin B12, vitamin C, vitamin D, vitamin E, vitamin K, and precursors thereof.

Examples of the fatty acids include oleic acid, arachidonic acid, and linoleic acid.

Examples of the lipids include cholesterols.

Examples of the inorganic ingredients include sodium, potassium, calcium, magnesium, phosphorus, and various trace elements (e.g., Co, Cu, F, Fe, Mn, Mo, Ni, Se, Si, Ni, Bi, V, and Zn). Specific examples of the inorganic ingredients include inorganic salts such as sodium chloride, potassium chloride, calcium chloride, magnesium sulfate, and sodium dihydrogenphosphate.

Examples of the pH-buffering agents include sodium hydrogencarbonate, phosphates, N,N-bis(2-hydroxyethyl)-2-aminoethanesulfonic acid (BES), 4-(2-hydroxyethyl)-1-piperazineethanesulfonic acid (HEPES), and N-[tris(hydroxymethyl)methyl]glycine (tricine).

Examples of the growth factors include fibroblast growth factor (FGF), hepatocyte growth factor (HGF), epidermal growth factor (EGF), transforming growth factor (TGF)-α, transforming growth factor (TGF)-β, vascular endothelial growth factor (VEGF), activin A, and insulin-like growth factor-1 (IGF-1).

Examples of the cytokines include interleukins.

Examples of the hormones include dexamethasone, hydrocortisone, estradiol, progesterone, glucagon, and insulin.

Examples of the cell adhesion factors or extracellular matrix components include type I collagen, type II collagen, fibronectin, laminin, poly-L-lysine, and poly-D-lysine.

Examples of the antibiotics include amphotericin B, kanamycin, gentamicin, streptomycin, and penicillin.

In one embodiment, the medium may be substantially free of serum. For example, the medium may be substantially free of serum during the entire culture period. The expression that "the medium is substantially free of serum" may mean that the serum content in the medium is 1% (w/w) or lower, 0.1% (w/w) or lower, 0.01% (w/w) or lower, or 0.001% (w/w) or lower, and may also mean that the serum content in the medium is 0 (zero) (i.e., the medium does not contain serum).

The various ingredients including the active ingredient may be contained in the starting medium, feed medium, perfusion medium, or a combination thereof. That is, the various ingredients including the active ingredient may be supplied to the medium individually or in appropriate combinations during the course of the culture. Any of these ingredients may be supplied once or multiple times or continuously. The compositions (e.g., types and/or concentrations of ingredients contained) of the starting medium, feed medium, and perfusion medium may or may not be the same. That is, the types of the ingredients contained in the starting medium may or may not be the same as the types of the ingredients contained in the feed medium or perfusion medium. The concentration of each ingredient contained in the starting medium may or may not be the same as the concentration of each corresponding ingredient contained in the feed medium or perfusion medium. For example, when the feed medium is used for the perfusion culture, the compositions of the starting medium and the feed medium may be the same. Further, two or more types of feed media or perfusion media with different compositions (e.g., types and/or concentrations of ingredients contained) may also be used. For example, when the feed medium or perfusion medium is supplied multiple times intermittently, the composition of the feed medium or perfusion medium may or may not be the same at each time. In addition, any of the various ingredients including the active ingredient may be supplied to the medium in a form that is not contained in the feed medium or perfusion medium, for example, in the form of powder,.

The inoculation amount of the animal cells at the start of the culture may be, for example, 1 × 10³ cells/mL or more, 1 × 10⁴ cells/mL or more, 1 × 10⁵ cells/mL or more, 1 × 10⁶ cells/mL or more, or 1 × 10⁷ cells/mL or more, and may be 1 × 10⁸ cells/mL or less, 1 × 10⁷ cells/mL or less, 1 × 10⁶ cells/mL or less, 1 × 10⁵ cells/mL or less, or 1 × 10⁴ cells/mL or less, in terms of viable cell count, or may be in a range defined by any non-contradictory combination of these minimum and maximum amounts. The inoculation amount of the animal cells at the start of the culture may specifically be, for example, 1 × 10³ to 1 × 10⁴ cells/mL, 1 × 10⁴ to 1 × 10⁵ cells/mL, 1 × 10⁵ to 1 × 10⁶ cells/mL, 1 × 10⁶ to 1 × 10⁷ cells/mL, or 1 × 10⁷ to 1 × 10⁸ cells/mL in terms of viable cell count. The inoculation amount of the animal cells at the start of the culture may specifically be, for example, 1 × 10³ to 1 × 10⁸ cells/mL, 1 × 10⁴ to 1 × 10⁷ cells/mL, or 1 × 10⁵ to 1 × 10⁶ cells/mL in terms of viable cell count. The viable cell count can be measured by using, for example, the Vi-CELL^{™} XR live/dead cell autoanalyzer (Beckman Coulter).

The culture may be performed, for example, in a CO₂-containing atmosphere such as 5 to 15% CO₂. The pH of the medium may be, for example, around neutral. The term "around neutral" may mean, for example, pH 6 to 8, pH 6.5 to 7.5, or pH 6.8 to 7.2. During the culture, the pH of the medium may be adjusted as needed. The pH of the medium can be adjusted by using various alkaline or acidic substances such as ammonia gas, aqueous ammonia, sodium carbonate, sodium bicarbonate, potassium carbonate, potassium bicarbonate, magnesium carbonate, sodium hydroxide, potassium hydroxide, calcium hydroxide, and magnesium hydroxide. The culture temperature may be, for example, 30 to 38°C. The culture period may be, for example, 0.5 days or longer, 1 day or longer, 2 days or longer, 3 days or longer, 4 days or longer, 5 days or longer, 6 days or longer, 7 days or longer, 8 days or longer, 9 days or longer, 10 days or longer, 12 days or longer, 15 days or longer, or 20 days or longer, and may be 60 days or shorter, 50 days or shorter, 40 days or shorter, 30 days or shorter, 25 days or shorter, 20 days or less, 15 days or less, 12 days or less, 10 days or less, 9 days or less, 8 days or less, or 7 days or less, or may be in a range defined by any non-contradictory combination of these minimum and maximum durations. The culture period may specifically be, for example, 1 to 60 days, 3 to 25 days, or 5 to 20 days. The culture may be continued, for example, until cultured meat is formed to a desired extent.

The concentration of the active ingredient in the medium may be, for example, 0.01 ng/mL or higher, 0.02 ng/mL or higher, 0.05 ng/mL or higher, 0.1 ng/mL or higher, 0.2 ng/mL or higher, 0.5 ng/mL or higher, 1 ng/mL or higher, 2 ng/mL or higher, 5 ng/mL or higher, 10 ng/mL or higher, 15 ng/mL or higher, 20 ng/mL or higher, or 25 ng/mL or higher, and may be 30 ng/mL or lower, 25 ng/mL or lower, 20 ng/mL or lower, 15 ng/mL or lower, 10 ng/mL or lower, 5 ng/mL or lower, 2 ng/mL or lower, 1 ng/mL or lower, 0.5 ng/mL or lower, 0.2 ng/mL or lower, 0.1 ng/mL or lower, 0.05 ng/mL or lower, or 0.02 ng/mL or lower, or may be in a range defined by any non-contradictory combination of these minimum and maximum concentrations. The concentration of the active ingredient in the medium may specifically be, for example, 0.01 to 0.02 ng/mL, 0.02 to 0.05 ng/mL, 0.05 to 0.1 ng/mL, 0.1 to 0.2 ng/mL, 0.2 to 0.5 ng/mL, 0.5 to 1 ng/mL, 1 to 2 ng/mL, 2 to 5 ng/mL, 5 to 10 ng/mL, 10 to 15 ng/mL, 15 to 20 ng/mL, 20 to 25 ng/mL, or 25 to 30 ng/mL. The concentration of the active ingredient in the medium may specifically be, for example, 0.01 to 30 ng/mL, 0.02 to 20 ng/mL, or 0.05 to 15 ng/mL.

All of the various ingredients including the active ingredient may be contained in the medium throughout the entire culture period, or may be contained in the medium during only a part of the culture period. That is, in order that "the culture is performed in a medium containing a certain ingredient" or "a certain ingredient is contained in the medium during the culture", it is sufficient if the ingredient is contained in the medium during at least a part of the culture period, and it is not necessary that the ingredient is contained in the medium throughout the entire culture period. All of the various ingredients including the active ingredient may be contained in the medium, for example, at the start of the culture, or may be supplied to the medium after the start of the culture. Further, all of the various ingredients including the active ingredient may be, for example, contained in the medium at the start of the culture and further supplied to the medium after the start of the culture (e.g., after consumption of the active ingredient).

All of the various ingredients including the active ingredient may be, for example, contained in the medium at any of the concentrations exemplified above throughout the entire culture period, or may be contained in the medium at any of the concentrations exemplified above during only a part of the culture period. That is, in order that "the culture is performed in a medium containing a certain ingredient at a certain concentration", "a certain ingredient is contained in the medium at the time of the culture at a certain concentration", or "concentration of a certain ingredient in the medium at the time of the culture is a certain concentration", it is sufficient that the concentration of the ingredient in the medium is within a range of that concentration during at least a part of the culture period, and it is not required that the concentration of the ingredient in the medium is within a range of that concentration throughout the entire culture period. All of the various ingredients including the active ingredient may be, for example, contained in the medium at any of the concentrations exemplified above at the start of the culture, or may be supplied to the medium so as to be contained at any of the concentrations exemplified above after the start of the culture. Further, all of the various ingredients including the active ingredient may be, for example, contained in the medium at any of the concentrations exemplified above at the start of culture, and further supplied to the medium so as to be contained at any of the concentrations exemplified above after the start of the culture (e.g., after consumption of the ingredients).

The length of the "part of the culture period" is not particularly limited as long as the growth-promoting effect can be obtained. The length of the "part of the culture period" can be appropriately set according to various conditions such as the type of animal cells and the length of the culture period. The "part of the culture period" may be, for example, 10% or more, 20% or more, 30% or more, 40% or more, 50% or more, 60% or more, 70% or more, 80% or more, 90% or more, 95% or more, 97% or more, or 99% or more of the total culture period. The "part of the culture period" may also be, for example, 10% or more, 20% or more, 30% or more, 40% or more, 50% or more, 60% or more, 70% or more, 80% or more, 90% or more, 95% or more, 97% or more, or 99% or more of the total period of the culture after the animal cells differentiate into myotube cells. The "part of the culture period" may also be, for example, a period of 0.5 days or longer, 1 day or longer, 2 days or longer, 3 days or longer, 4 days or longer, 5 days or longer, 6 days or longer, 7 days or longer, 8 days or longer, 9 days or longer, 10 days or longer, 12 days or longer, or 15 days or longer.

The concentrations of all of the various ingredients including the active ingredient in the medium may be, for example, set to any of the concentrations exemplified above as an average value during a specific period of the culture. That is, the expression that "the culture is performed in a medium containing a certain ingredient at a certain concentration", "a certain ingredient is contained in the medium at the time of the culture at a certain concentration", or "concentration of a certain ingredient in the medium at the time of culture is a certain concentration" may mean that the average value of the concentration of the ingredient in the medium over a specific period during the culture is within a range of that concentration. The "average value of the concentration of an ingredient in the medium during a specific period of the culture" is not particularly limited as long as the variation of the concentration of the ingredient during the specific period of the culture can be grasped, but, for example, it may mean an average value of the concentrations of the ingredient in the medium measured every 60 minutes, 30 minutes, 20 minutes, or 10 minutes during the specific period of the culture. Examples of the "specific period of the culture" include the entire culture period and a part of the culture period. The "part of the culture period" is as explained above.

All of the various ingredients including the active ingredient may be supplied to the medium throughout the entire culture period or during only a part of the culture period. The "part of the culture period" is as described above. All of the various ingredients including the active ingredient may be supplied to the medium, for example, continuously or intermittently. All of the various ingredients including the active ingredient may be supplied to the medium, for example, daily or every few days.

The concentrations of all of the various ingredients including the active ingredient in the feed medium or perfusion medium may be, for example, within any of the ranges of the concentration of the ingredient in the medium exemplified above. The concentrations of the various ingredients including the active ingredient in the feed medium or perfusion medium may be, for example, not lower than 1 time, not lower than 1.1 times, not lower than 1.3 times, not lower than 1.5 times, not lower than 2 times, not lower than 3 times, not lower than 5 times, not lower than 7 times, not lower than 10 times, not lower than 15 times, or not lower than 20 times, and may be not higher than 100 times, not higher than 70 times, not higher than 50 times, not higher than 30 times, not higher than 20 times, not higher than 15 times, not higher than 10 times, not higher than 7 times, not higher than 5 times, not higher than 3 times, or not higher than 2 times, of any of the concentrations of the ingredients in the medium exemplified above, or may be in a range defined by any non-contradictory combination of these minimum and maximum concentrations. The concentrations of the various ingredients including the active ingredient in the feed medium or perfusion medium may specifically be, for example, 1 to 2 times, 1.1 to 2 times, 1.3 to 2 times, 1.5 to 2 times, 2 to 3 times, 3 to 5 times, 5 to 7 times, 7 to 10 times, 10 to 15 times, 15 to 20 times, 20 to 30 times, 20 to 50 times, 20 to 70 times, or 20 to 100 times of any of the concentrations of the ingredients in the medium exemplified above. The concentrations of the various ingredients including the active ingredient in the feed medium or perfusion medium may specifically be, for example, 1 to 100 times, 2 to 50 times, or 5 to 20 times of any of the concentrations of the ingredients in the medium exemplified above.

The concentrations of all of the various ingredients including the active ingredient can be measured by, for example, known methods used for detection or identification of compounds. Examples of such methods include HPLC, UPLC, LC/MS, GC/MS, and NMR.

By culturing animal cells as described above, cultured meat can be obtained.

### EXAMPLES

Hereafter, the present invention will be is explained more specifically with reference to non-limiting examples.

### Reference Example 1: Construction of Corynebacterium glutamicum deficient in O-mannosyltransferase gene pmt1

### (1) Construction of vector pBSSTΔpmt1 for deletion of pmt1 gene

It is known that O-glycosylation occurs in secreted proteins expressed by *C. glutamicum* as the host, and that deletion of the protein O-mannosyltransferase PMT1 can eliminate the ability to modify secreted proteins by O-glycosylation (Martina Mahne et al., The *Corynebacterium glutamicum* gene pmt encoding a glycosyltransferase related to eukaryotic protein-O-mannosyltransferases is essential for glycosylation of the resuscitation promoting factor (Rpf2) and other secreted proteins. FEMS Microbiol Lett. 2006 Jun;259(2):226-33).

The genome sequence of the *C. glutamicum* ATCC 13869 strain and the nucleotide sequence of the pmt1 gene encoding protein O-mannosyltransferase PMT1 have already been determined (GenBank Accession No. AP017557, NCBI locus_tag CGBL_0109730). The nucleotide sequence of the pmt1 gene of the *C. glutamicum* ATCC 13869 strain and the amino acid sequence of PMT1 encoded by the gene are shown as SEQ ID NOS: 39 and 40, respectively.

By PCR using chromosomal DNA of the *C. glutamicum* ATCC 13869 strain prepared by using the PurElute^{™} Genomic DNA Kit (EdgeBio) as a template, and the primers of SEQ ID NOS: 41 and 42 or the primers of SEQ ID NOS: 43 and 44, a region of approximately 1 kbp upstream sequence on the 5' side of the pmt1 gene and a region of approximately 1 kbp downstream sequence on the 3' side of the pmt1 gene were amplified, respectively. Then, by PCR using the both amplified DNA fragments as templates and DNAs shown as SEQ ID NOS: 41 and 44 as primers, a DNA fragment of approximately 2 kbp, which consisted of the both DNA fragments fused, was obtained. For the PCR, Pyrobest^{®} DNA Polymerase (Takara Bio) was used, and the reaction conditions were according to the protocol recommended by the manufacturer. This DNA fragment was inserted into pBS5T described in WO2006/057450 at the SmaI site to obtain a vector pBSSTΔpmt1 for deletion of the pmt1 gene. For the ligation reaction, DNA Ligation Kit Mighty Mix (Takara Bio) was used, and the reaction conditions were according to the protocol recommended by the manufacturer.

### (2) Construction of pmt1 gene-deficient strain of C. glutamicum YDK0107 strain

The *C. glutamicum* YDK0107 strain described in WO2016/171224 was transformed with pBSSTΔpmt1 constructed in (1). The *C. glutamicum* YDK0107 strain is a natural mutant strain obtained from the *C. glutamicum* YDK010 strain as a parent strain, in which a W302C mutation is introduced into the phoS gene (WO2016/171224). The *C. glutamicum* YDK010 strain is a cell surface layer protein CspB-deficient strain of *C. glutamicum* AJ12036 (FERM BP-734) (WO2002/081694). From the resulting transformants, a strain was selected according to the method described in WO2006/057450 to obtain the YDK0107Δpmt1 strain, which is deficient in the pmt1 gene.

### Example 1: Secretory expression of bovine basic fibroblast growth factor (bbFGF) by Corynebacterium glutamicum

### (1-1) Construction of plasmid for secretory expression of bbFGF with TorA signal sequence

The amino acid sequence of the bovine basic fibroblast growth factor-2 (hereafter referred to as bbFGF) has already been determined (UniProt Accession No. P03969). This amino acid sequence is shown as SEQ ID NO: 46.

Considering the codon usage frequency in *C. glutamicum*, a nucleotide sequence encoding bbFGF was designed. The designed nucleotide sequence is shown as SEQ ID NO: 45.

Then, a fusion protein (TorAss-bbFGF) of bbFGF with the 39 amino acid residues of the signal peptide of the TorA protein derived from *E. coli* (UniProt Accession No. P33225) was designed to enable secretory expression of bbFGF by *C. glutamicum.* The nucleotide sequence encoding the designed TorAss-bbFGF and the amino acid sequence thereof are shown as SEQ ID NOS: 47 and 48, respectively, Further, an expression cassette of TorAss-bbFGF consisting of the nucleotide sequence encoding TorAss-bbFGF, and the promoter of the cspB gene derived from the *C. glutamicum* ATCC 13869 strain and ligated upstream of the nucleotide sequence, as well as KpnI site added on the 5'-side, and ApaI site added on the 3'-side was designed, and the entire sequence thereof was synthesized.

The expression cassette of TorAss-bbFGF was inserted into pPK10 described in WO2021/112249 at the KpnI-ApaI site to construct pPK10_TorAss-bbFGF, which is a secretory expression plasmid for bbFGF utilizing the TorA signal sequence. Nucleotide sequencing of the inserted fragment confirmed that the expression cassette of TorAss-bbFGF was constructed as designed. The nucleotide sequencing was performed by using BigDye^{®} Terminator v3.1 Cycle Sequencing Kit (Applied Biosystems) and 3500xL Genetic Analyzer (Applied Biosystems).

### (1-2) Secretory expression of bbFGF in C. glutamicum

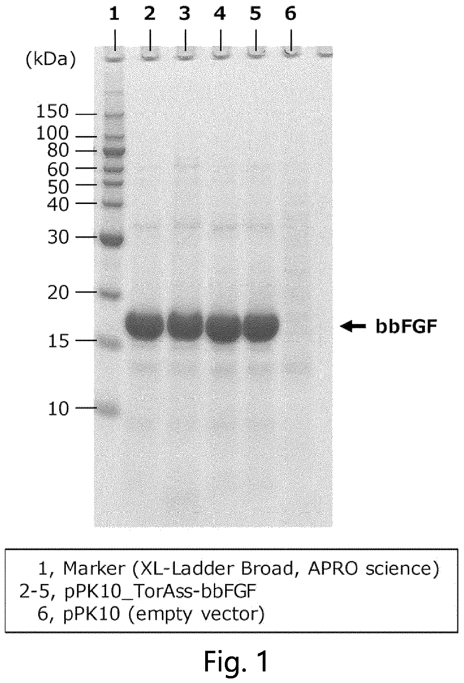

The *C. glutamicum* YDK0107Δpmt1 strain described in Reference Example 1 was transformed by using pPK10_TorAss-bbFGF to obtain a YDK0107Δpmt1/pPK10_TorAss-bbFGF strain. The obtained transformants were cultured in MMTG liquid medium (prepared by dissolving 120 g of glucose, 3 g of magnesium sulfate heptahydrate, 30 g of ammonium sulfate, 1.5 g of potassium dihydrogenphosphate, 0.03 g of iron sulfate heptahydrate, 0.03 g of manganese sulfate pentahydrate, 0.45 mg of thiamine hydrochloride, 0.45 mg of biotin, 0.15 g of DL-methionine, soy beans hydrochloric acid hydrolysis solution (total nitrogen 0.2 g), and 50 g of calcium carbonate in water to a volume of 1 L and adjusting pH to 7.0) containing 25 mg/L kanamycin at 30°C for 72 hours. After the completion of the culture, 6.5 µL of the culture supernatant obtained by centrifugation of each culture medium was subjected to reducing SDS-PAGE using NuPAGE^{®} 12% Bis-Tris Gel (Thermo Fisher Scientific), and then staining was performed with Quick-CBB (Wako). As a result, a protein band presumed to be bbFGF was detected in the culture supernatant of the YDK0107Δpmt1/pPK10 _TorAss-bbFGF strain (Fig. 1, Lanes 2 to 5).

### Example 2: Evaluation of biological activity of bovine basic fibroblast growth factor (bbFGF) secreted by Corynebacterium glutamicum (1)

### (2-1) Preparation of bbFGF crude purified fraction A

The *C. glutamicum* YDK0107Δpmt1/pPK10 _TorAss-bbFGF strain was cultured in a fermenter according to a known method (Hiroshi Itaya and Yoshimi Kikuchi. Secretion of *Streptomyces mobaraensis* pro-transglutaminase by coryneform bacteria. Appl Microbiol Biotechnol. 2008; 78(4): 621-625). The centrifugation supernatant of the culture medium was diluted 6.6 times with ultrapure water and sterile filtered through a 0.2 µm PVDF membrane filter. This filtrate was designated as crude purified fraction A and cryopreserved at -80°C until evaluation of the biological activity.

### (2-2) Measurement of activity of bbFGF crude purified fraction A

The activity of the crude purified fraction A obtained above was measured as cell proliferation-promoting activity by using BALB/c 3T3 cells. As a reference sample for the activity, the international standard of human-derived bFGF (WHO International Standard FGF-2, Human, rDNA-derived, NIBSC code 90/712, specific activity 8.0 × 10⁵ U/mg) was used. The concentration of bbFGF in the crude purified fraction A was determined by reverse phase HPLC quantification using bFGF (basic FGF) Solution, Human, Recombinant (154 a.a.), Animal-Free (Nacalai Tesque, Cat # 19155-07) as a reference sample. As a result, the cell proliferation-promoting activity value (specific activity) of the crude purified fraction A was 3.6 × 10⁶ U/mg.

### Example 3: Evaluation of biological activity of bovine basic fibroblast growth factor (bbFGF) secreted by Corynebacterium glutamicum (2)

### (3-1) Preparation of bbFGF crude purified fraction B

The *C. glutamicum* YDK0107Δpmt1/pPK10 _TorAss-bbFGF strain was cultured in a fermenter according to the known method (Hiroshi Itaya and Yoshimi Kikuchi. Secretion of *Streptomyces mobaraensis* pro-transglutaminase by coryneform bacteria. Appl Microbiol Biotechnol. 2008; 78(4): 621-625). To the centrifugation supernatant of the culture medium, dithiothreitol was added at a concentration of 1.03 mg/mL. After standing for 15 minutes, the supernatant of the culture medium was diluted 6.6 times with ultrapure water and sterile filtered through a 0.2 µm PVDF membrane filter. This filtrate was designated as crude purified fraction B and cryopreserved at -80°C until evaluation of the biological activity.

### (3-2) Measurement of activity of bbFGF crude purified fraction B

The activity of the crude purified fraction B obtained above was measured as cell proliferation-promoting activity by using the BALB/c 3T3 cells. As a reference sample for the activity, the international standard of human-derived bFGF (WHO International Standard FGF-2, Human, rDNA-derived, NIBSC code 90/712, specific activity 8.0 × 10⁵ U/mg) was used. The concentration of bbFGF in the crude purified fraction B was determined by reverse phase HPLC quantification using bFGF (basic FGF) Solution, Human, Recombinant (154 a.a.), Animal-Free (Nacalai Tesque, Cat # 19155-07) as a reference sample. As a result, the cell proliferation-promoting activity value (specific activity) of the crude purified fraction B was 2.5 × 10⁶ U/mg.

### Example 4: Evaluation of biological activity of bovine basic fibroblast growth factor (bbFGF) secreted by Corynebacterium glutamicum (3)

### (4-1) Preparation of bbFGF crude purified fraction C

The *C. glutamicum* YDK0107Δpmt1/pPK10 _TorAss-bbFGF strain was cultured in a fermenter according to the known method (Hiroshi Itaya and Yoshimi Kikuchi. Secretion of *Streptomyces mobaraensis* pro-transglutaminase by coryneform bacteria. Appl Microbiol Biotechnol. 2008; 78(4): 621-625). To the centrifugation supernatant of the culture medium, dithiothreitol was added at a concentration of 1.03 mg/mL. After standing for 15 minutes, solvent substitution of the supernatant of the culture medium was performed with a solution prepared by adding dithiothreitol to PBS (Thermo Fisher Scientific, Cat # 14190) at a concentration of 154 mg/L by using an ultrafiltration membrane of molecular weight cut-off 3000. The solvent-substituted supernatant was sterile filtered with a 0.22 µm PVDF membrane. This filtrate was designated as crude purified fraction C and cryopreserved at -80°C until evaluation of the biological activity.

### (4-2) Measurement of activity of bbFGF crude purified fraction C

The activity of the crude purified fraction C obtained above was measured as cell proliferation-promoting activity by using the BALB/c 3T3 cells. As a reference sample for the activity, the international standard of human-derived bFGF (WHO International Standard FGF-2, Human, rDNA-derived, NIBSC code 90/712, specific activity 8.0 × 10⁵ U/mg) was used. The concentration of bbFGF in the crude purified fraction C was determined by reverse phase HPLC quantification using bFGF (basic FGF) Solution, Human, Recombinant (154 a.a.), Animal-Free (Nacalai Tesque, Cat # 19155-07) as a reference sample. As a result, the cell proliferation-promoting activity value (specific activity) of the crude purified fraction C was 3.1 × 10⁶ U/mg.

### Example 5: Evaluation of biological activity of bovine basic fibroblast growth factor (bbFGF) secreted by Corynebacterium glutamicum (4)

### (5-1) Preparation of bbFGF crude purified fraction D

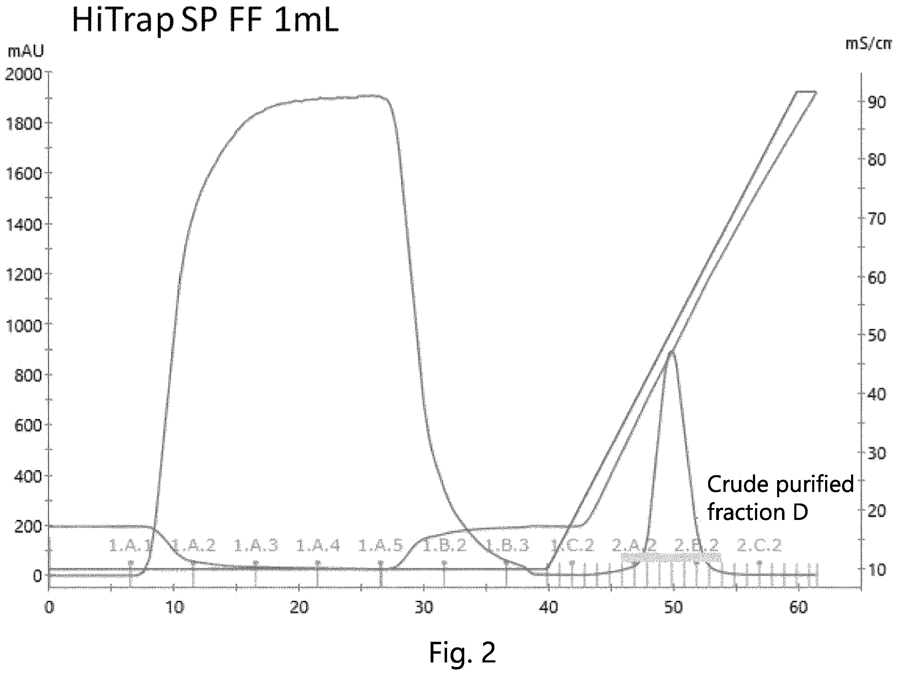

The *C. glutamicum* YDK0107Δpmt1/pPK10 _TorAss-bbFGF strain was cultured in a fermenter according to the known method (Hiroshi Itaya and Yoshimi Kikuchi. Secretion of *Streptomyces mobaraensis* pro-transglutaminase by coryneform bacteria. Appl Microbiol Biotechnol. 2008; 78(4): 621-625). The centrifugal supernatant of the culture medium was adjusted to pH 6.5 using 50% acetic acid, and then dithiothreitol (DTT) was added and dissolved at a concentration of 27 mg/mL. After standing for 15 minutes, water was added to the supernatant to attain a conductivity of 9.5 mS/cm (25°C). The supernatant was filtered through a 0.2 µm PVDF membrane filter, and the filtrate was used as the loading solution for cation exchange chromatography. As the column, HiTrap SP FF (Cytiva Cat# 17504501) was used, and after it was equilibrated with A-buffer (a solution prepared by adding 908 mL of water to 5.7 g of disodium hydrogenphosphate, 11.7 g of sodium dihydrogenphosphate, 0.15 g of DTT, and 5.4 g of sodium chloride), and the aforementioned loading solution was loaded. After re-equilibration with A-buffer, elution was performed with a gradient to B-buffer (0 to 100% of B-buffer, 20 CV, B-buffer is a solution prepared by adding 1000 mL of water to 3.6 g of disodium hydrogenphosphate, 0.15 g of DTT, and 6.0 g of sodium chloride, adjusting the resulting solution to pH 8.0 with addition of A-buffer, and adding sodium chloride to a concentration of 1 M) (Fig. 2). The peak fractions were collected as crude purified fraction D and cryopreserved at -80°C until evaluation of the biological activity.

### (5-2) Measurement of activity of bbFGF crude purified fraction D

The activity of the crude purified fraction D obtained above was measured as cell proliferation-promoting activity by using the BALB/c 3T3 cells. As a reference sample for the activity, the international standard of human-derived bFGF (WHO International Standard FGF-2, Human, rDNA-derived, NIBSC code 90/712, specific activity 8.0 × 10⁵ U/mg) was used. The concentration of bbFGF in the crude purified fraction D was determined by reverse phase HPLC quantification using bFGF (basic FGF) Solution, Human, Recombinant (154 a.a.), Animal-Free (Nacalai Tesque, Cat # 19155-07) as a reference sample. As a result, the cell proliferation-promoting activity value (specific activity) of the crude purified fraction D was 2.3 × 10⁶ U/mg.

### Example 6: Evaluation of biological activity of bovine basic fibroblast growth factor (bbFGF) secreted by Corynebacterium glutamicum (5)

### (6-1) Preparation of bbFGF crude purified fraction E

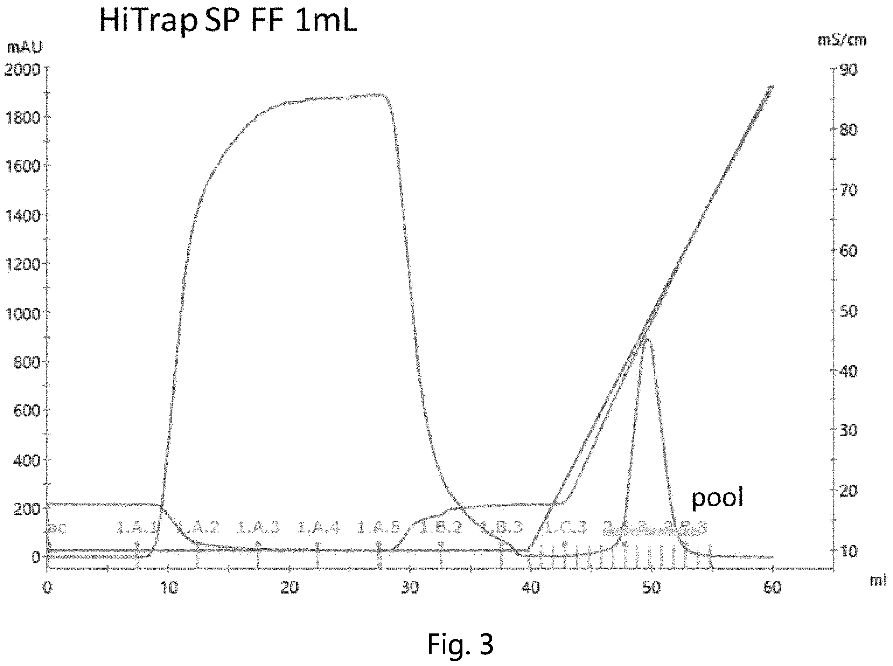

The *C. glutamicum* YDK0107Δpmt1/pPK10_TorAss-bbFGF strain was cultured in a fermenter according to the known method (Hiroshi Itaya and Yoshimi Kikuchi. Secretion of *Streptomyces mobaraensis* pro-transglutaminase by coryneform bacteria. Appl Microbiol Biotechnol. 2008; 78(4): 621-625). The centrifugal supernatant of the culture medium was adjusted to pH 6.5 using 50% acetic acid, and dithiothreitol (DTT) was added and dissolved at a concentration of 27 mg/mL. After standing for 15 minutes, water was added to the supernatant to attain a conductivity of 9.5 mS/cm (25°C). The supernatant was filtered through a 0.22 µm PVDF membrane filter, and the filtrate was used as the loading solution for cation exchange chromatography. As the column, HiTrap SP FF (Cytiva Cat# 17504501) was used, and after it was equilibrated with A-buffer (a solution prepared by adding 908 mL of water to 5.7 g of disodium hydrogenphosphate, 11.7 g of sodium dihydrogenphosphate, 1.7 g of EDTA, and 5.4 g of sodium chloride), and the aforementioned loading solution was loaded. After re-equilibration with A-buffer, elution was performed with a gradient to B-buffer (0 to 100% of B-buffer, 20 CV, B-buffer is a solution obtained by adding 1000 mL of water to 3.6 g of disodium hydrogenphosphate, 1.9 g of EDTA, and 6.0 g of sodium chloride, and adding sodium chloride to the resulting solution to a concentration of 1 M) (Fig. 3). The peak fractions (Fig. 3, pool) were collected, and subjected to solvent substitution with a solution prepared by adding dithiothreitol to PBS (Thermo Fisher Scientific, Cat # 14190) at a concentration of 154 mg/L using an ultrafiltration membrane of molecular weight cut-off 3000. The solvent-substituted fractions were sterile filtered with a 0.22 µm PVDF membrane. This filtrate was designated as crude purified fraction E and cryopreserved at -80°C until evaluation of the biological activity.

### (6-2) Measurement of activity of bbFGF crude purified fraction E

The activity of the crude purified fraction E obtained above was measured as cell proliferation-promoting activity by using the BALB/c 3T3 cells. As a reference sample for the activity, the international standard of human-derived bFGF (WHO International Standard FGF-2, Human, rDNA-derived, NIBSC code 90/712, specific activity 8.0 × 10⁵ U/mg) was used. The concentration of bbFGF in the crude purified fraction E was determined by reverse phase HPLC quantification using bFGF (basic FGF) Solution, Human, Recombinant (154 a.a.), Animal-Free (Nacalai Tesque, Cat # 19155-07) as a reference sample. As a result, the cell proliferation-promoting activity value (specific activity) of the crude purified fraction E was 2.6 × 10⁶ U/mg.

As shown in Examples 2 to 6, all the fractions showed high cell proliferation-promoting activity values. That is, it was revealed that bbFGF produced by using a coryneform bacterium as an expression host can be suitably used for culturing animal cells (specifically, for production of cultured meat). In particular, it was revealed that bbFGF produced by using a coryneform bacterium as an expression host can be suitably used for animal cell culture (specifically, for production of cultured meat) without purification to a high purity with chromatography or the like.

### Example 7: Evaluation of biological activity of bovine basic fibroblast growth factor (bbFGF) secreted by Corynebacterium glutamicum (6)

### (7-1) Preparation of bovine muscle stem cells

Muscle tissues were collected from edible bovine shanks, and CD56-positive cells were isolated by using a magnetic activated cell separation system (MACS) and used as bovine muscle stem cells.

### (7-2) Evaluation of bbFGF activity using bovine muscle stem cells in serum-free medium

The bovine muscle stem cells obtained above were suspended in a serum medium. As the serum medium, Advanced DMEM (Thermo Fisher Scientific, Cat No. 12491015) supplemented with 20% FBS (Thermo Fisher Scientific, Cat No. 10270106), 10% horse serum (Thermo Fisher Scientific, Cat No. 16050-130), and 1% GlutaMAX^{™} Supplement (Thermo Fisher Scientific, Cat No. 35050061) was used. The cell suspension was inoculated on a 6-well plate (BD FALCON, Cat # 353046) coated with 1 µg/cm² of fibronectin (Corning, Cat # F2006) at a cell density of 5300 cells/2 mL/cm², and the medium was changed every other day. On day 4 of the culture, the cells were subcultured in a bbFGF-free and serum-free medium or bbFGF-containing serum-free medium, and the medium was changed every other day. As the bbFGF-free and serum-free medium, StemFit^{R} Basic03 (Ajinomoto Co., Ltd.) supplemented with 5 ng/mL of HGF (Peprotech, Cat # 294HGN005), and 0.5 mg/mL of Soy protein acid hydrolysate (Sigma, Cat # S1674) was used. As the bbFGF-containing serum-free medium, the bbFGF-free and serum-free medium supplemented with the bbFGF crude fraction A, bbFGF crude fraction E, or Animal-Free Recombinant Bovine FGF-basic (Peprotech, Cat # AF-450-62) at a concentration of 0.1, 1, or 10 ng/mL was used. The group numbers and media are summarized in Table 1. Cell counting and subculture were performed every 4 days, and growth rate and doubling time during the two consecutive subcultures of from day 4 to day 12 were calculated.

**Table 1.**

| Group No. | Medium | bbFGF addition concentration |
|---|---|---|
| 3 | bbFGF-free and serum-free medium | No addition |
| 4 | bbFGF-containing serum-free medium | 0.1 ng/mL Animal-Free Recombinant Bovine FGF-basic |
| 5 | bbFGF-containing serum-free medium | 1 ng/mL Animal-Free Recombinant Bovine FGF-basic |
| 6 | bbFGF-containing serum-free medium | 10 ng/mL Animal-Free Recombinant Bovine FGF-basic |
| 7 | bbFGF-containing serum-free medium | 0.1 ng/mL bbFGF crude purified fraction A |
| 8 | bbFGF-containing serum-free medium | 1 ng/mL bbFGF crude purified fraction A |
| 9 | bbFGF-containing serum-free medium | 10 ng/mL bbFGF crude purified fraction A |
| 10 | bbFGF-containing serum-free medium | 0.1 ng/mL bbFGF crude purified fraction E |
| 11 | bbFGF-containing serum-free medium | 1 ng/mL bbFGF crude purified fraction E |
| 12 | bbFGF-containing serum-free medium | 10 ng/mL bbFGF crude purified fraction E |

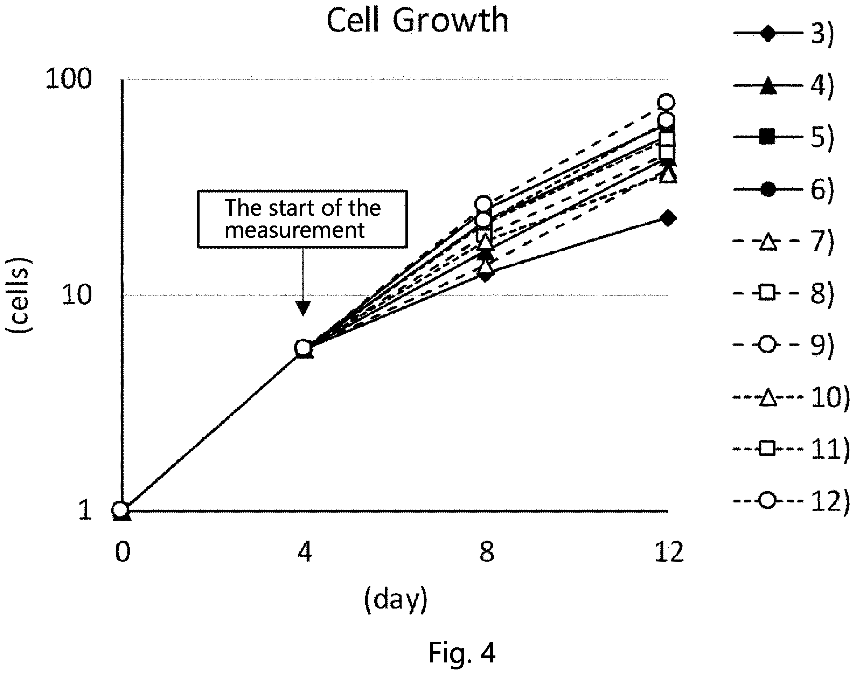

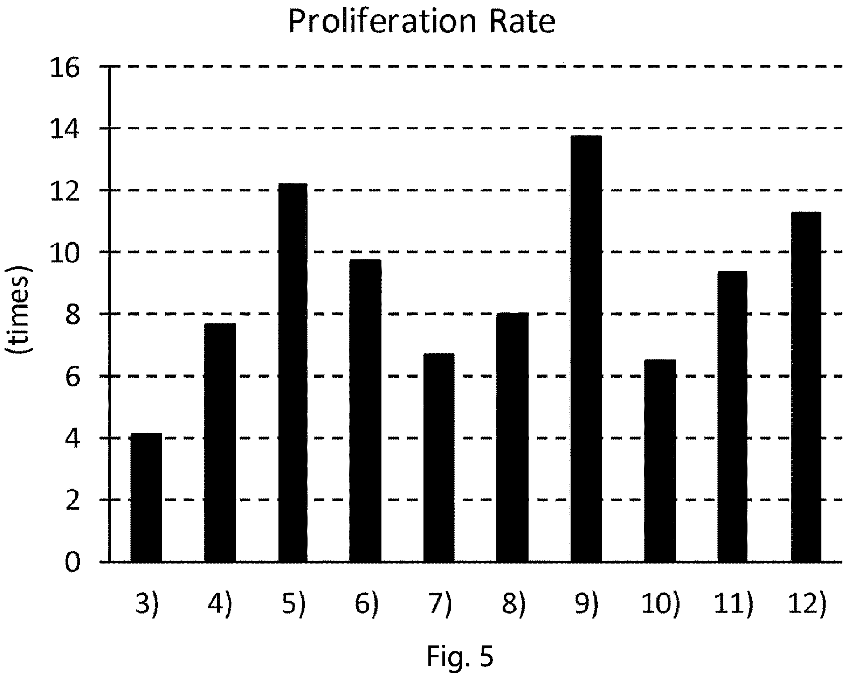

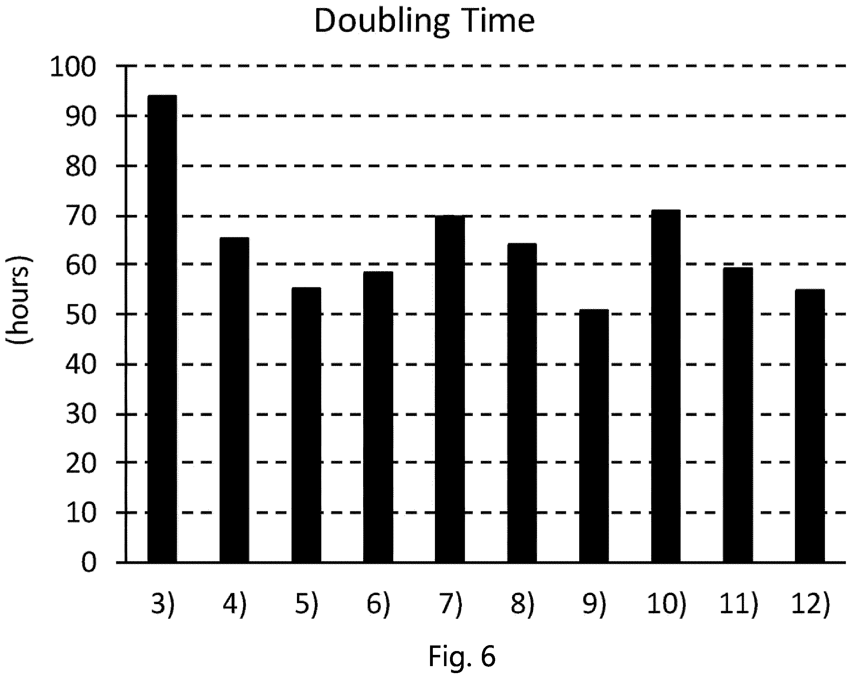

The results are shown in Figs. 4 to 6. All of the bbFGF crude fraction A, bbFGF crude fraction E, and Animal-Free Recombinant Bovine FGF-basic promoted proliferation of bovine muscle stem cells in the serum-free medium. That is, it was revealed that bbFGF produced by using a coryneform bacterium as an expression host can be suitably used for animal cell culture (specifically, production of cultured meat). In particular, it was revealed that bbFGF produced by using a coryneform bacterium as an expression host can be suitably used for animal cell culture (specifically, production of cultured meat) without purification to a high purity with chromatography or the like.

### Example 8: Evaluation of biological activity of bovine basic fibroblast growth factor (bbFGF) secreted by Corynebacterium glutamicum (7)

### (8-1) Preparation of bovine muscle stem cells

Muscle tissues were collected from edible bovine shanks, and CD56-positive cells were isolated by using a magnetic activated cell separation system (MACS) and used as bovine muscle stem cells.

### (8-2) Evaluation of bbFGF activity using bovine muscle stem cells

The bovine muscle stem cells obtained above were suspended in a serum medium. As the serum medium, Advanced DMEM (Thermo Fisher Scientific, Cat No. 12491015) supplemented with 20% FBS (Thermo Fisher Scientific, Cat No. 10270106), 10% horse serum (Thermo Fisher Scientific, Cat No. 16050-130), and 1% GlutaMAX^{™} Supplement (Thermo Fisher Scientific, Cat No. 35050061) was used. The cell suspension was inoculated on a 6-well plate (BD FALCON, Cat # 353046) coated with 1 µg/cm² of fibronectin (Corning, Cat # F2006) at a cell density of 5300 cells/2 mL/cm², and the medium was changed every other day. On day 4 of the culture, the cells were subcultured in a bbFGF-free and serum-free medium or bbFGF-containing serum-free medium, and the medium was changed every other day. As the bbFGF-free and serum-free medium, StemFit^{®} Basic03 (Ajinomoto Co., Ltd.) supplemented with 5 ng/mL of HGF (Peprotech, Cat # 294HGN005), and 0.5 mg/mL of Soy protein acid hydrolysate (Sigma, Cat # S1674) was used. As the bbFGF-containing serum-free medium, the bbFGF-free and serum-free medium supplemented with the bbFGF crude fraction B, bbFGF crude fraction C, bbFGF crude fraction D, or Animal-Free Recombinant Bovine FGF-basic (Peprotech, Cat #AF-450-62) at a concentration of 0.1, 1, or 10 ng/mL was used. The group numbers and media are summarized in Table 2. The cell suspension was inoculated on a 6-well plate (BD FALCON, Cat # 353046) coated with 1 µg/cm² of fibronectin (Corning, Cat # F2006) at a cell density of 5300 cells/2 mL/cm², and the medium was changed every other day. Cell counting and subculture were performed every 4 days, and growth rate and doubling time during the two consecutive subcultures of from day 4 to day 12 were calculated.

**Table 2.**

| Group No. | Medium | bbFGF addition concentration |
|---|---|---|
| 1 | bbFGF-free and serum-free medium | No addition |
| 2 | bbFGF-containing serum-free medium | 0.1 ng/mL Animal-Free Recombinant Bovine FGF-basic |
| 3 | bbFGF-containing serum-free medium | 1 ng/mL Animal-Free Recombinant Bovine FGF-basic |
| 4 | bbFGF-containing serum-free medium | 10 ng/mL Animal-Free Recombinant Bovine FGF-basic |
| 5 | bbFGF-containing serum-free medium | 0.1 ng/mL bbFGF crude purified fraction B |
| 6 | bbFGF-containing serum-free medium | 1 ng/mL bbFGF crude purified fraction B |
| 7 | bbFGF-containing serum-free medium | 10 ng/mL bbFGF crude purified fraction B |
| 8 | bbFGF-containing serum-free medium | 0.1 ng/mL bbFGF crude purified fraction C |
| 9 | bbFGF-containing serum-free medium | 1 ng/mL bbFGF crude purified fraction C |
| 10 | bbFGF-containing serum-free medium | 10 ng/mL bbFGF crude purified fraction C |
| 11 | bbFGF-containing serum-free medium | 0.1 ng/mL bbFGF crude purified fraction D |
| 12 | bbFGF-containing serum-free medium | 1 ng/mL bbFGF crude purified fraction D |
| 13 | bbFGF-containing serum-free medium | 10 ng/mL bbFGF crude purified fraction D |

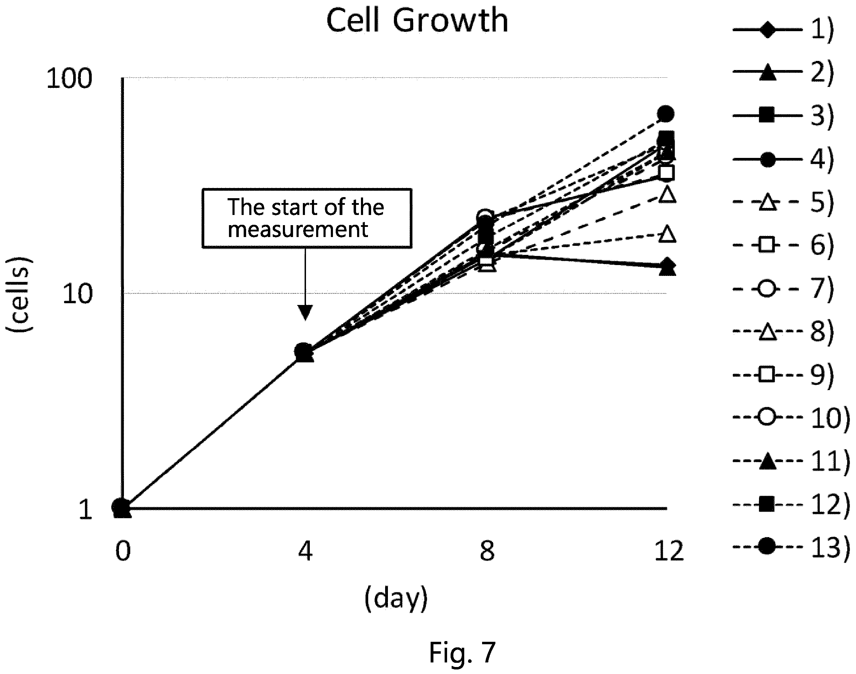

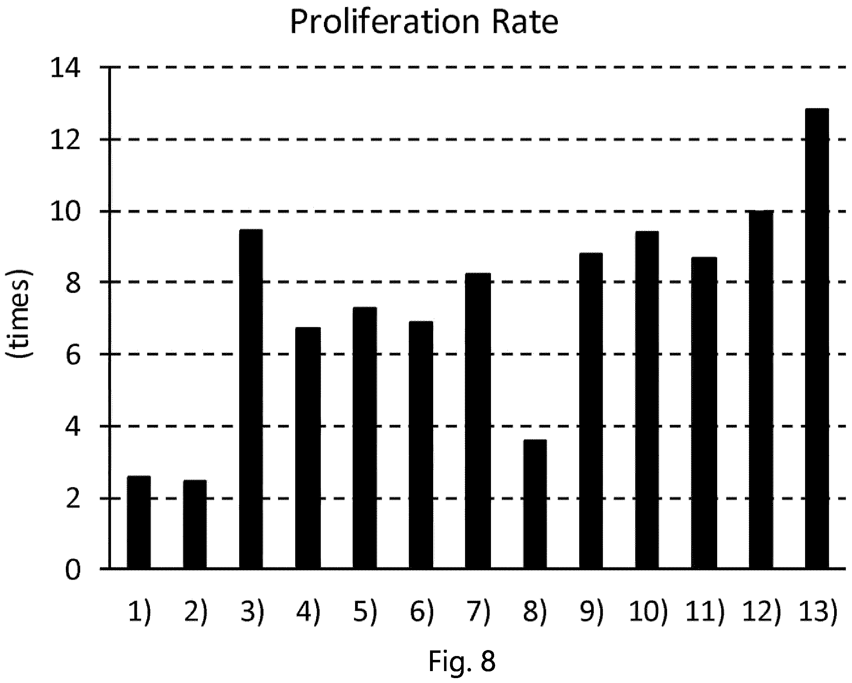

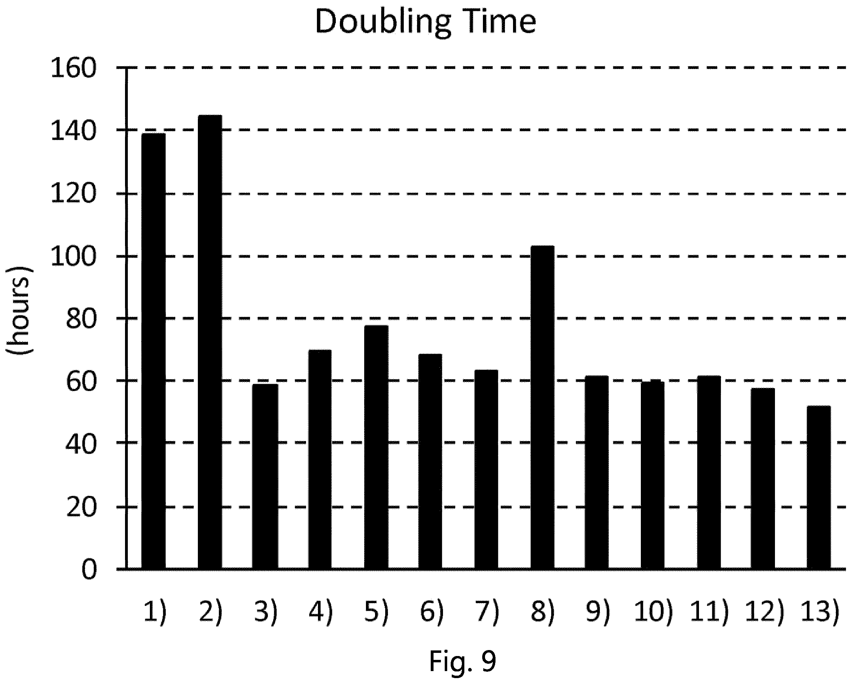

The results are shown in Figs. 7 to 9. All of the bbFGF crude fraction B, bbFGF crude fraction C, bbFGF crude fraction D, and Animal-Free Recombinant Bovine FGF-basic promoted proliferation of bovine muscle stem cells in the serum-free medium. That is, it was revealed that bbFGF produced by using a coryneform bacterium as an expression host can be suitably used for animal cell culture (specifically, production of cultured meat). In particular, it was revealed that bbFGF produced by using a coryneform bacterium as an expression host can be suitably used for animal cell culture (specifically, production of cultured meat) without purification to a high purity with chromatography or the like.

### INDUSTRIAL AVAILABILITY

According to the present invention, cultured meat can be efficiently produced.

### Explanation of Sequence Listing

SEQ ID NO: 1: Nucleotide sequence of the phoS gene of *C. glutamicum* YDK010
SEQ ID NO: 2: Amino acid sequence of the PhoS protein of *C. glutamicum* YDK010
SEQ ID NO: 3: Amino acid sequence of the PhoS protein of *C. glutamicum* ATCC 13032
SEQ ID NO: 4: Amino acid sequence of the PhoS protein of *C. glutamicum* ATCC 14067
SEQ ID NO: 5: Amino acid sequence of the PhoS protein of *C. callunae*
SEQ ID NO: 6: Amino acid sequence of the PhoS protein of *C. crenatum*
SEQ ID NO: 7: Amino acid sequence of the PhoS protein of *C. efficiens*
SEQ ID NO: 8: Nucleotide sequence of the phoR gene of *C. glutamicum* ATCC 13032
SEQ ID NO: 9: Amino acid sequence of the PhoR protein of *C. glutamicum* ATCC 13032
SEQ ID NO: 10: Nucleotide sequence of the cspB gene of *C. glutamicum* ATCC 13869
SEQ ID NO: 11: Amino acid sequence of the CspB protein of *C. glutamicum* ATCC 13869
SEQ ID NO: 12: Nucleotide sequence of the tatA gene of *C. glutamicum* ATCC 13032
SEQ ID NO: 13: Amino acid sequence of the TatA protein of *C. glutamicum* ATCC 13032
SEQ ID NO: 14: Nucleotide sequence of the tatB gene of *C. glutamicum* ATCC 13032
SEQ ID NO: 15: Amino acid sequence of the TatB protein of *C. glutamicum* ATCC 13032
SEQ ID NO: 16: Nucleotide sequence of the tatC gene of *C. glutamicum* ATCC 13032
SEQ ID NO: 17: Amino acid sequence of the TatC protein of *C. glutamicum* ATCC 13032
SEQ ID NO: 18: Amino acid sequence of the TorA signal peptide
SEQ ID NO: 19: Amino acid sequence of the SufI signal peptide
SEQ ID NO: 20: Amino acid sequence of the PhoD signal peptide
SEQ ID NO: 21: Amino acid sequence of the LipA signal peptide
SEQ ID NO: 22: Amino acid sequence of the IMD signal peptide
SEQ ID NO: 23: Amino acid sequence of the twin-arginine motif
SEQ ID NO: 24: Skipped sequence
SEQ ID NO: 25: Amino acid sequence of the PS1 signal peptide
SEQ ID NO: 26: Amino acid sequence of the PS2 signal peptide
SEQ ID NO: 27: Amino acid sequence of the SlpA signal peptide
SEQ ID NO: 28: Amino acid sequence of the CspB mature protein of *C. glutamicum* ATCC 13869
SEQ ID NOS: 29 to 31: Skipped sequences
SEQ ID NOS: 32 to 36: Amino acid sequences of one embodiment of the insertion sequence used in the present invention
SEQ ID NO: 37: Recognition sequence of the Factor Xa protease
SEQ ID NO: 38: Recognition sequence of the ProTEV protease
SEQ ID NO: 39: Nucleotide sequence of the PMT gene of *C. glutamicum* ATCC 13869
SEQ ID NO: 40: Amino acid sequence of the PMT protein of *C. glutamicum* ATCC 13869
SEQ ID NOS: 41 to 44: Primers
SEQ ID NO: 45: Nucleotide sequence of the bbFGF gene
SEQ ID NO: 46: Amino acid sequence of bbFGF
SEQ ID NO: 47: Nucleotide sequence for TorAss-bbFGF
SEQ ID NO: 48: Amino acid sequence of TorAss-bbFGF

## Claims

1. A method of producing bovine basic fibroblast growth factor (bbFGF) for production of cultured meat, the method comprising:
a step of culturing a coryneform bacterium having a gene construct for secretory expression of bbFGF to obtain a culture product containing bbFGF.

2. The method according claim 1, further comprising a step of removing cells of the coryneform bacterium from the culture product.

3. The method according to claim 1 or 2, not comprising a step of purifying bbFGF.

4. The method according to any one of claims 1 to 3, not comprising a step of purifying bbFGF with chromatography.

5. The method according to any one of claims 1 to 4, wherein the gene construct contains a promoter sequence that functions in the coryneform bacterium, a nucleic acid sequence encoding a signal peptide that functions in the coryneform bacterium, and a nucleic acid sequence encoding bbFGF in the direction from 5' to 3', and
wherein the bbFGF is expressed as a fusion protein with the signal peptide.

6. The method according to any one of claims 1 to 5, wherein the coryneform bacterium is a bacterium belonging to the genus *Corynebacterium.*

7. The method according to any one of claims 1 to 6, wherein the coryneform bacterium is *Corynebacterium glutamicum.*

8. A method of producing cultured meat, comprising:
a step of culturing animal cells in the presence of bovine basic fibroblast growth factor (bbFGF),
wherein the bbFGF has been produced by the method according to any one of claims 1 to 7.
